# EUROPEAN PATENT APPLICATION

(11) **EP 2 567 972 A1**
(43) Date of publication of application: **13.03.2013**
(21) Application number: 11180891.1
(22) Date of filing: 12.09.2011
(51) Int. Cl.: C07K 16/08, G01N 33/53

(54) **Antibodies against E7 protein of Human Papilloma Virus (HPV)**

(71) Applicant: Adriacell S.p.A., 34149 Trieste (IT)
(72) Inventor: Kuehne, Christian, 1050 Vienna (AT)
(74) Representative: Sonn & Partner Patentanwälte

(57) **Abstract**

The invention discloses a method for preparing Human Papilloma Virus protein E7 antigen (HPV E7 antigen) comprising the following steps:
- providing a purified preparation of HPV protein E7,
- phosphorylating the HPV protein E7 in the preparation,
- purifying the phosphorylated E7 protein with an anion exchange chromatography, wherein the phosphorylated E7 protein is separated from the non-phosphorylated E7 protein by a step wherein the non-phosphorylated E7 protein stays bound to an anion exchanger during the anion exchange chromatography whereas the phosphorylated E7 protein is obtained in the eluate of the anion exchange chromatography, thereby
- obtaining a purified preparation of a phosphorylated HPV protein E7 antigen.

The invention further discloses antibodies specific to this antigen, a kit and method for using such antibodies in clinical diagnostics and methods for generation of such antibodies.

## Description

The present invention relates to antibodies to E7 protein of Human Papilloma Virus (HPV) and to methods of preparation and provision of HPV E7 proteins

HPV are small, non-enveloped circular DNA double strand viruses. The episomal viral genome is approximately 8 kilodaltons (kDa) in size. The small size of the HPV genome results in a limited number of virally encoded proteins and functions. These proteins contain all the necessary information to ensure the fidelity of the entire viral life cycle and, as such, are a direct indicator of viral activity at any stage in infected host cells. This feature classifies the viral proteins as ideal and well-accepted targets for diagnostic, predictive, preventive and therapeutic interventions.

Most HPV types contain only eight ORFs (open reading frames) that are all transcribed from one DNA strand. ORFs are divided into the early (E) and late (L) region encoded proteins. The E proteins (E1, E2, E4, E5, E6, E7) are all necessary and essential for viral replication. The L proteins (L1, L2) constitute the components of the virion and function in virion assembly.

The E1 and E2 proteins encode for "viral specificity factors" which recruit and direct host cell effector proteins for viral transcription and replication. These proteins constitute the HPV-specific viral origin recognition complex and are essential factors for initiation of DNA replication from the HPV viral origin. In addition, E2 is a key regulator of viral transcription activation and repression from the virus-specific early and late promoters.

The E4 and E5 proteins are less well understood. Whereas E4 is mainly attributed to late state functions, E5 plays a role in both early and late state functions during the viral life cycle. The E6 and E7 proteins (and to a lesser extent E4 and E5) encode for the "viral surveillance" proteins and interfere with a limited number of negative and positive cellular key regulatory proteins in order to create a host cell environment that is permissive for viral replication. To do this, E6 proteins target the p53 surveillance pathway and the E7 protein targets the p105Rb surveillance pathway through various direct or indirect interactions. E6 and E7 facilitate stable maintenance of viral episomes and interact with cellular factors to cause differentiating cells to re-enter S-phase.

The L1 and L2 proteins assemble into the viral icosahedral capsomers that form around the viral genome to generate infective virus particles.

The viral life cycle is not fully understood, mainly due to the lack of proteomic tools that allow direct molecular analysis of the virally encoded proteins from clinical specimens during viral replication. Many arguments are based on surrogate markers and DNA and/or RNA analysis. Current understanding is that HPVs are selectively epitheliotropic and establish productive infections only inside stratified epithelia such as the anogenital tract, the oral cavity and the skin. For a complete viral life cycle that brings about infective HPV virus particles, differentiation of the infected basal epithelial cells is essential. During infection, the virus targets the basal squamous epithelial host cell receptors and is internalized by vesicle transport. After vesicle release the virus is transported to the nucleus and subsequently the viral DNA is uncoated. Early viral proteins are produced in the basal cells that enable specific initiation of viral DNA replication, which is at this stage under host cell licensing control, producing one episomal viral DNA double-strand genome per basal cell. Following the differentiation of the basal epithelial cells into cells which no longer undergo cellular DNA synthesis, unlicensed multiple episomal viral DNA synthesis and the production of the late proteins for virus capsomer formation takes place. This results in the production of active virions that are released after nuclear breakdown.

Persistent high-risk HPV infection is the causative agent in more than 98% of malignancies of the uterine cervix. HPV is a sexually transmitted disease with high infection rates but low disease progression. Most of the infections are transient and tend to resolve without treatment, becoming undetectable within 1 to 2 years. In 2002, cancer of the cervix uteri resulted in 274,000 deaths and an estimated 493,000 new cases were reported. In developing countries, cervical cancer accounts for 15% of female cancers with a risk of 1.5 % for women under the age of 65. In developed countries, it accounts for only 3.6 % of female cancers with a risk of 0.8 % for women under the age of 65. Besides cancer of the cervix uteri, a 20 to 80% incidence rate of HPV-induced cancers are observed for cancers of the penis, vulva, anus and some cancers of the oropharynx, including the middle of the throat containing the soft palate, the base of the tongue and the tonsils. There is increasing evidence that HPV might also be the causative agent for the carcinogenesis of subpopulations of additional cancer indications.

More than 100 types of HPVs have been identified. Around 40 of them infect the genital tract (mucosal and cutaneous lesions) and are taxonomically classified as the Alpha-papillomavirus genus. HPVs which infect skin (cutaneous lesions) belong to the Beta-papillomavirus genus. A total of 16 different papillomavirus genera can be distinguished.

From the Alpha-papillomavirus genus, there is sufficient evidence in humans for 12 HPV types as causative agents of carcinogenicity (high-risk HPV). According to the International Agency for Research and Cancer (IARC), these include HPV51 of the species 5, HPV56 of species 6, HPVs 18, 39, 45, 59 of species 7, and HPV 16, 31, 33, 35, 52, 58 of species 9. HPV66 from the species 6 is still under discussion.

The most prominent type of HPV associated with Alpha-papillomavirus-induced malignancies is HPV16 and together with HPV18, the second most frequent type, represents more than 70% of all the squamous cell carcinomas (SCC) and more than 85% of the adenocarcinomas (ADC) of the cervix. HPV16 alone is associated with more than 86% of the HPV-positive oropharyngeal SCCs.

High-risk virus infection alone does not necessarily cause progression to cervical cancer and only a small percentage of precursor lesions of infected individuals precede cancer development with latency periods of 10 to 20 years. The immune system is essential for control of HPV infections and individuals with impaired immune systems often exhibit persistent infections. Regression is associated with both the humoral and cellular immune response. It has been suggested that modification of specific cellular genes during the course of tumour progression may occur although this remains controversial. However, it is known that malignant transformation is only initiated if a persistent high-risk virus loses its replication competence. This occurs if the episomal viral DNA becomes integrated into the host cell DNA, which is observed mainly for high grade lesions. During this recombination process, substantial parts of the viral genome are frequently deleted and this renders the virus inactive (non-replicative). Whereas most of the virus-encoded ORFs are dispensable for malignancy, the E6 and E7 genes must be functionally conserved during this process. At this stage E6 and E7 can be transcribed from nearby cellular promoters.

Few to multiple copies of truncated viral DNA are found in the genomes of HPV-induced cancer cells that are competent to produce functional E6 and E7 proteins. Both proteins have to be continuously expressed for carcinogenesis. As such, HPV-induced cancer contains only small remnants of the HPV virus, namely E6 and E7. Recent genome-wide analysis of tumour specimens has confirmed that HPV-induced cancers have two hallmarks: the chromosomal integration of functional E6 and E7 genes and no or only very late upcoming mutations in critical tumour suppressor and oncogenic pathways. The elimination of the major viral components makes preventive vaccination for progressive tumour stages non-functional and explains the limited specificity of DNA/RNA testing.

It is now well accepted in the field that HPV-induced tumour induction, progression and maintenance is caused by the virus-encoded oncoproteins E6 and E7. As described above, these proteins are essential for regulatory functions during the viral life cycle. When most other viral proteins are inactivated or lost and no complete viral replication with consequent host cell lysis occurs, the various high-risk specific E6 and E7 proteins gain transforming function through interactions with vital cellular surveillance proteins such as p53 or p105RB. In all experimental systems E7 has been shown to be the prominent oncoprotein, exhibiting stronger malignant transforming effects than E6. Given the essential causal function for all stages of HPV-induced carcinogenesis, E7 makes an ideal target for diagnosis. As such, the presence of E7 proteins in human specimen samples directly predicts malignancy and cancer. It is important to note that epithelial cells that do not contain detectable E7 protein but may have HPV DNA are either regressing or not progressing in carcinogenesis.

Cervical cancer screening is the biggest single global oncology diagnostic market. The in vitro diagnostic (IVD) market is currently valued at $40 bn and has been growing at 6.7% annually. However, certain tests, such as cervical cancer tests, are growing at a rate of 14% annually.

The first line test is cytological analysis (PAP smear), a highly successful early tumour diagnostic test. The PAP test has recently been adapted for liquid sampling (liquid PAP smear). The PAP test has high specificity with medium sensitivity, with the reliability of the test strongly dependent the level of medical expertise. False negative rates can be as high as 50%. Although this test has been used for many decades and is followed with subsequent colposcopic interventions, the decrease rate of cervical squamous cell carcinoma has reached a plateau. Cervical adenocarcinomas are barely detected by PAP testing.

For the staging of precursors to cervical cancer, intraepithelial neoplasia biopsies are analyzed by the pathologist. The World Health Organisation classification and its modification are currently applied for biopsy screening in the pathology field. The term Cervical Intraepithelial Neoplasia (CIN) is used to classify different stages: CIN1 for mild dysplasia, CIN2 for moderate dysplasia CIN3 for severe dysplasia and carcinoma in situ (CIS). More recently, CIN1 and CIN2 as well as CIN2 and CIN3 have been combined into CIN1/CIN2 and CIN2/3, respectively. For PAP smear cytology, the Bethesta System is mainly used. The terms Low-grade Squamous Intraepithelial Lesion (LSIL) and High-grade Squamous Intraepithelial Lesion (HSIL) are used. LSIL PAP corresponds mainly to CIN1 dysplasia and HSIL indicates CIN2/CIN3. The new term ASC-H is used to denote atypical cells at higher-risk of association with precancerous atypical squamous cells of undetermined significance and ASCUS denotes cytological atypical lesions that cannot be classified as normal but are difficult to attribute to the LSIL/HSIL system.

As a supporting test to the PAP test, HPV molecular diagnostics testing has been introduced. DNA and RNA tests are now used to define the HPV risk factor for cervical cancer. DNA testing is either based on the Hybrid Capture technique (Qiagen, formerly Digene) or on the PCR amplification reaction (Roche). In addition, RNA testing of the viral oncogenes has recently been launched ("Nuclisense EASY nQ" bio-Mèrieux). Specimens are mainly from liquid PAP sampling. These molecular diagnostic methods show excellent sensitivity (low false negative rates) but very low prognostic value for the disease. The latter is mainly due to the fact that HPV infections are persistent for years with no progression into a neoplastic state (>10 years). It is important to note that viral mRNA levels do not reflect the actual protein expression levels or pathology.

There is unmet need for early and specific diagnosis of HPV caused malignant pathology for early therapeutic intervention and therapy follow-up. The now generally accepted central essential requirement of a causal presence of high risk E7 proteins in all stages of HPV caused disease induction, progression and maintenance classifies these proteins as superior diagnostic or prognostic biomarker for HPV induced cancers. It is intended that suitable E7 antibodies are promising diagnostic tools for primary as well as adjunctive HPV caused disease screening. So far E7 proteins are difficult to access for proper antibody production, due to their biological, highly toxic immunosuppressive nature and their biochemical features such as their primary and secondary structural properties.

E7 proteins are highly negatively charged polypeptides with a predicted molecular weight in the range of 11 kDa. Interestingly, all the different E7 types migrate aberrantly in SDS polyacrylamide gels in the range of 15 to 21 kDa due to their highly anionic nature. By their sequence homology conservation and functional similarity with adenovirus (Ad) E1A and simian vacuo-lating virus 40 (SV40) large tumour antigen (T Ag) the amino-terminal part is divided into the CR1 (conserved region 1) and CR2 homology domain. These two conserved regions significantly contribute to the malignant transforming activity of the high risk E7 protein types. CR2 contains the essential high affinity consensus binding site Leu-X-Cys-X-Glu for pRb and pRb like proteins and the adjacent Casein Kinase phosphorylation site motif, hallmark sequence features of all the high risk type E7 proteins. The E7 carboxy-terminal part (CR3) exists of a zinc-binding domain spanning two Cys-X-X-Cys motifs. High resolution 3-dimensional structure studies from recombinant E7 protein preparations show that the E7 carboxy-terminal part forms a unique, rigid and tightly packed zinc-binding fold and most important that the amino-terminal part is structurally unfolded and highly flexible and as such not well defined in these preparations (McLoughlin-Drubin et al., Virology 384 (2009), 335-344).

Antibodies against HPV protein E7 have been disclosed in the prior art (see e.g. WO 03/080669 A2, WO 2011/101122 A1). Antigens for antibody preparations were either E7 specific sequence
containing fragments such as peptides, recombinant truncated E7 proteins, full-length recombinant E7 or recombinant E7 fusion proteins. Antibodies obtained so far can be divided into two classes: antibodies interacting with linear epitopes obtained from linear peptide antigens or the E7 amino-terminal antigens, and antibodies interacting with the zinc-binding fold that are mainly interacting with structural epitopes from the rigid zinc-binding fold. Application for diagnostic of the antibodies from both of these two classes are very limited and in particular for analysing gynaecological PAP samples in immunocytochemistry and cyto-fluorimetry as well as biopsies in immunohistochemistry. Linear epitopes are known to show low affinity and/or specificity resulting frequently in no or only weak signals in these diagnostic tests. In contrary epitopes against rigid structures such as zinc-binding domains, although well suited for liquid tests with non-denatured, native antigens, need epitope retrieval bringing about complete refolding in situ in clinical specimens. The quality of the latter procedure greatly influences the sensitivity, quantity and distribution of the signal showing positivity, and thus these procedures are barely independent of inter-observer-variability in the clinics. Given the fact that virtually all clinical diagnostic tests use denaturation protocols for standardization of sampling and sample preparation these antibodies depend on validated well standardized, time and material consuming refolding (antigen retrieval) procedures. It is this distinction what is the mayor object of the invention. In the case of the invention E7 proteins are produced with recombinant techniques and post-expression modification that brings about E7-antigens with specifically ordered amino-terminal folding properties, which introduces antibodies interacting and recognizing the linear and in the same time the folded antigen. The antibodies of the invention all are reacting with denatured as well as native proteins with similar affinities. When these antibodies are used with completely or partially unfolded E7 proteins, as is the case in the majority of the clinical diagnostic samples, it is the nature of the antibodies that fold the denatured proteins resulting in a high affinity and specificity interaction, ideally suited for the said diagnostic assays. In applying a priori tightly packed rigid E7 carboxy-terminal antigens including zinc-binding folds, does not provide the necessary flexibility for a structure refolding caused by the antibody.

Suitable preparation of sufficient amounts of native, natural E7 antigens from clinical samples or cell culture of cancer cells that could be used as antigens for antibody production, is so far not convincingly achieved. This is mainly due to the fact that E7 forms part of difficult to handle high molecular weight complexes *in vivo.* In addition in-purification analytics was very difficult so far, due to the lack of suitable antibodies. Molecular biological methods to express individual HPV antigens from any HPV type redefined the approach to produce HPV antibodies. Especially bacterial fusion proteins had several advantages: they provided an inexpensive, plentiful and reproducible source of E7 viral antigens from any HPV type. The main disadvantage was that most fusion proteins are insoluble and had to be used in Western blot assays under denaturing conditions that provide only linear epitopes. A series of type-specific antibodies have been generated from HPV recombinant proteins expressed in different heterologous systems. These antibodies can be used to demonstrate the expression of recombinant E7 proteins in experimental sample cells, mainly were recombinant E7 proteins are expressed from heterologous promoters. These experimental systems are well known in the field to provide artificial overproduction of E7 proteins with different activity and localisation behaviour than endogenous E7 molecules from cancer cells and the interpretation and conclusive discussions are very limited. The latter is of particular interest as many of the arguments for cellular localisation for E7 are based on these types of experiments. However most of these antibodies available do not recognize native E7 from clinical samples in a reproducible and robust way and thus their use is mostly restricted to in-vitro research assays. There were reports that a polyclonal rabbit antiserum raised by immunization with highly purified recombinant E7 proteins and subsequent affinity purification against the same antigen was was used to detect E7 by immunohistochemical staining of paraffin sections of biopsies of cervical HSIL and cervical cancer tissues.

On the other hand, human-serum antibodies to E6 and E7 proteins of HPV infected individuals and from HPV induced cancers are only detected in less than 50% of late stage tumour patients. Moreover the quality of the serum antibodies is of very low affinity and all exhibit low specificity. Most relevant stages for diagnostics such as CIN1 to CIN3 barely produce serum antibodies, which is explained by the B and T cell immunosuppressive nature of the E7 proteins and their use as biomarker for a late stage therapy follow up is not suitable.

The use of E6 and E7 antibodies for the diagnosis of HPV induced cancer was already apparent in initial studies, were only linear epitope recognizing antibodies by either peptide ELISA or Western blot analysis were used despite the low sensitivity and specificity of these assays. Methods that apply full-length E7 proteins that present conformational epitopes, i.e. immunoprecipitation assays with in-vitro transcribed and translated HPV 16 E7 proteins showed higher sensitivity and specificity (ELISAs that use yeast-expressed biochemically purified and renatured full-length HPV 16 and 18 E7 proteins have been shown to be even more specific and equally sensitive compared with radioimmunoprecipitation assays), yet still not in a manner to allow a proper and reliable HPV protein E7 detection method for biological samples in routine testing so that sill no epidemiological studies using such assays have yet been published.

E7 and to a lesser extend E6 protein detection is generally accepted as the theoretically ideal biomarker for diagnosis of high risk HPV induced disease. However by the fact that no suitable E7 antibodies were available so far for routine testing other early biomarker were analysed that could act as an adjunct to current cytological and histological assessment of cervical smears and biopsies with the final goal to identify those individuals that have ambiguous results and decide on potential treatment (Tsoumpou et al., Cancer Treat Rev., 35 (2009), 210-220).

The E7-regulated p16INK4A tumour suppressor is the most advanced marker used as a surrogate for E7 function and, as such, indirectly defining tumourigenesis of cervical cancer. E7 releases the pRb/E2F transcription repressor complex thus enabling G1 cell cycle release and the progression into S phase of the cell cycle. Moreover p16INK4A a CDK4/5 inhibitor is stabilized which acts similar as a loss of function by this the p16INK4A is accumulated in E7 positive cells. Using available suitable p16 antibodies increase in p16INK4A protein can be detected as a biomarker for active malignant cells. Although there is good evidence that p16INK4A correlates with the severity of malignant abnormalities in cytology and biopsies the reproducibility is limited due to the difficulty in standardization of the test. The latter is mainly due to inter-observer variability by the fact that p16INK4A in CIN1 and CIN2 is not always detected as a homogenous pattern, moreover it is also expressed, although usually to a lower extend, in non E7 containing cells and a general consensus establishing threshold values above which a sample is positive for p16INK4A is needed for interpretation. It is expected from a diagnostic test that can adjunct or outperform a PAP testing as primary screening test that inter-observer variability is limited to a minimum. Finally some individuals with HPV induced malignant disease show p16INK4A null mutations.

One of the big advantages of the E7 biomarker is that there is no cellular background if the cells are not infected with HPV and also staining clearly is homogenously detectable from CIN1 to CIN3 as well.

Although p16INK4A assays show significantly higher prognostic value for the disease, therapy follow-up is only limited applicable. In addition for late stage, many chemotherapeutics currently used for the treatment of cancer, such as cisplatin which is used for cervical cancer therapy, induce p16 and other CDK inhibitors independently of HPVE7 which further limits the general application of such assays.

It is an object of the present invention to improve HPV induced disease diagnostics. It is a specific object to provide suitable HPV diagnostic means to be combined with or replace current diagnostic standards, such as the PAP test. It is a further object to provide improved E7 antibodies with a higher specificity and sensitivity compared to known E7 antibodies. This should enable improved HPV/(cervical) cancer testing which is also reliably useable in routine testing providing a sensitive and specific tool for improving diagnostics, prognostics and therapy follow up in this field.

Therefore, the present invention provides a method for preparing Human Papilloma Virus protein E7 antigen (HPV E7 antigen) comprising the following steps:
- providing a purified preparation of HPV protein E7,
- phosphorylating the HPV protein E7 in the preparation,
- purifying the phosphorylated E7 protein with an anion exchange chromatography, wherein the phosphorylated E7 protein is separated from the non-phosphorylated E7 protein by a step wherein the non-phosphorylated E7 protein stays bound to an anion exchanger during the anion exchange chromatography whereas the phosphorylated E7 protein is obtained in the eluate of the anion exchange chromatography, thereby
- obtaining a purified preparation of a phosphorylated HPV protein E7 antigen.

With the method according to the present invention, a completely unique HPV E7 antigen is provided which enables the generation of a new class of E7-specific antibodies. The antibodies generated with the novel antigen according to the present invention enable for the first time a HPV diagnostic analysis which can be applied to all cellular assays, especially to PAP smears. The antibodies generated with the novel antigens enable the allocation and detection of functional E7 proteins, especially in the nucleus. This was specifically surprising, since the E7 antigen was known to be immune suppressive. With the novel antigen according to the present invention, antibodies of all kinds, especially monoclonal antibodies can easily be generated. Obviously, up to the present invention, provision of a real native form of the physiologically/pathologically active HPV E7 protein was not possible; this is why antibodies were regarded as being very difficult to produce and, if production was achieved in principle, were not useable for a reliable detection of E7 antigen, especially these prior art antibodies were not able to homogenously detect the functional E7 antigen in the nucleus (see e.g. WO 03/080669 A2; Dreier et al., Virology 409 (2011), 54-68, WO 2011/101122 A1 and McLoughlin et al., 2009. Also detection in Western blots was very unreliable and therefore not useable in the diagnostic practice (see above). With the present method, the novel E7 antigen can be produced from various suitable sources, e.g. as recombinant protein, preferably as a recombinant protein produced in E.coli, B. subtilis, S. cerevisiae P. pastoris, insect cells, plant cells, especially A.thaliana, N. tabacum or O. sativa or a mammalian host cell line, especially HEK293, CHO-K1 or CHO-WBL cells.

The purified preparation of HPV protein E7 to be used as a starting material for the present method should contain the E7 protein in purified form so as to allow proper phosphorylation. This means that substances which disturb phosphorylation should be kept at a minimum level. Also other proteins, which could compete with the E7 protein for phosphorylation should preferably prevented by prior E7 purification. Since the E7 protein, especially produced recombinantly is known very well in the present field, there is no problem to provide suitable purified E7 material for the present method. According to a preferred embodiment of the present method, the purified preparation of HPV protein E7 contains more than 50 % HPV protein E7 per total protein, preferably more than 70 % HPV protein E7 per total protein, especially more than 90 % HPV protein E7 per total protein.

Phosphorylation is preferably performed at the optimum conditions for the kinase with suitable amounts of HPV protein E7. For example, the phosphorylation can be performed at a protein concentration of 10 µg to 100 mg E7 protein per ml, preferably of 0.05 to 10 mg E7 protein per ml, especially of 0.1 to 5 mg E7 protein per ml.

It is necessary for the further use of the antigen according to the present invention, especially for using the antigen for generating antibodies, that the phosphorylation is performed to a significant extent. The E7 protein to be used has to be fully phosphorylated. This makes it necessary that a significant amount of the total preparation contains fully phosphorylated E7 protein. According to a preferred embodiment, the phosphorylation is therefore performed until at least 40%, preferably at least 80 %, especially at least 90 % of the E7 protein is fully phosphorylated. This can be done and monitored by usual techniques in order to establish and optimise the phosphorylation details.

In principle, any kinase that is able to phosphorylate the E7 protein can be used according to the present invention. According to a preferred embodiment of the present invention, phosphorylation is performed with a Casein Kinase (EC 2.7.11.1), preferably with Casein Kinase I or Casein Kinase II, especially recombinant Casein Kinase II, Casein Kinase II from rat liver or Casein Kinase II from calf thymus. These kinases are known to phosphorylate E7 protein *in vivo* but up to now efficient phosphorylation of E7 by these kinases could not be achieved yet in vitro. Moreover, up to now, the use of such aimed phosphorylation for providing the antigen according to the present invention e.g. for vaccination purposes or for the generation of anti-E7-antibodies was not suggested in the art.

The phosphorylation can be performed e.g. at a temperature of 15 to 40°C, preferably from 20 to 37°C, and for a time of 10 min to 24 h, preferably from 30 min to 12 h, especially from 1 to 6 h.

The decisive step for generating the novel antigen preparation according to the present invention is - besides a complete phosphorylation - the separation of the phosphorylated form of E7 from the non-phosphorylated by anion exchange chromatography. Surprisingly, it was possible to conduct the anion exchange chromatography in a way that the fully phosphorylated form is not bound to the anion exchanger whereas the E7 forms which are not or not fully phosphorylated are bound to the anion exchanger. With this anion exchanger method, it was possible to separate both forms and to obtain a preparation of fully phosphorylated E7 antigen which enables the production of novel HPV vaccines and novel anti-HPV-E7 antibodies with surprising properties, specifically for detection of E7 protein in biological samples.

According to a preferred embodiment of the method according to the present invention, the phosphorylated HPV E7 protein is subjected to the anion exchange chromatography in a buffer having a ionic strength of 400mM NaCl or less, preferably of 250 mM NaCl or less, especially of 150 mM NaCl or less.

Preferred anion exchange chromatography methods are performed with a beaded agarose or sepharose column with positively charged side chains, preferably a diethylaminoethyl, a quaternary aminoethyl or a quaternary ammonium agarose column, especially a Q Sepharose, MonoQ Sepharose CIM (Convective Interaction Media)-QA, CIM-DEAE or DEAE Sepharose anion exchange column.

According to a preferred embodiment of the present invention, the purified preparation of the phosphorylated HPV protein E7 antigen is dialysed, preferably at a temperature of 6 to 30°C, especially dialysed in the presence of Trehalose.

According to a specifically important aspect, the present invention is drawn to a HPV E7 antigen preparation wherein the HPV E7 protein is fully phosphorylated and is essentially free of contaminating nucleic acids and/or lipids, specifically to a preparation which is essentially free from nucleic acids. It has been realised with the present invention that presence of nucleic acids can significantly disturb antibody generation for E7, because of the binding properties of E7 to nucleic acids. It is evident that previous E7 antibody preparations have a bias with respect to this issue, because the E7 protein seems to have a different confirmation, depending whether it is bound to nucleic acids or not (the same might hold true for lipids). Accordingly, the present antigen preparation is free from nucleic acids, thereby displaying the E7 protein in a form without the nucleic acid-bound conformation.

This is specifically important if the HPV E7 antigen preparation is a recombinantly produced protein. The recombinantly produced E7 protein may be any relevant form of E7 from any HPV strain; the wild type forms as well as the mutants known in the art; all these proteins can be used according to the present invention, provided that they still contain the phosphorylation sites.

Preferably, processing tools can be provided in form of additional amino acid sequences to enable improved handling of the recombinant protein. For example, His-tags or other sequences may be added to the HPV E7 sequence. It is therefore preferred, if the E7 antigen contains a His-tag, a methionine residue, a serine residue at the N-terminus or at the C-terminus of the natural E7 protein sequence, especially a MHHHHHHS-peptide, a MHHHHHHSMSENLYFQGS-peptide or a GS-peptide at the N-terminus.

Virtually all HPV E7 antigens can be prepared by the method according to the present invention (again, provided that the separation concerning the phosphorylated/not (fully) phosphorylated form is possible), a preferred HPV E7 protein is selected from HPV 16, 18, 31, 33, 35, 39, 45, 51, 52, 56, 58, 59 and 66, preferably HPV 16, 18, 31, 33 and 45, especially HPV 16 and 18 (HPV amino acid and nucleic acid sequences are given in SEQ.ID.NOs.21 to 24).

A preferred use of the novel antigen provided by the present invention is the use as a vaccine. The vaccine can be any form of protein E7; however, E7 mutants lacking p105Rb binding are specifically preferred due to the potentially oncogenic nature of the wild type E7 forms. Suitable examples of such mutants are disclosed in McLoughlin et al., 2009. The fully phosphorylated antigen according to the present invention can be provided as an efficient vaccine. Therefore, it is preferred to provide the present HPV E7 antigen preparation with additional vaccine substances, e.g. an adjuvant, preferably complete or incomplete Freund's adjuvant, aluminum salts (alum), especially aluminum hydroxide, aluminum phosphate, and aluminum sulphate; 3-De-O-acylated monophosphoryl lipid A (MPL), MF59, AS03, AS04, polysorbate 80 (Tween 80), imidazopyridine compounds, imidazoquinoxaline compounds, saponins, especially QS21; oil in water emulsions, especially comprising squalene or peanut oil; optionally in combination with immune stimulants, especially monophosphoryl lipid A, CpG, polymeric or monomeric amino acids, especially polyglutamic acid, polyarginine or polylysine. The HPV E7 antigen may also be coupled to suitable carriers, e.g. a protein carrier, such as KLH (Keyhole Limpet Hemocyanin), tetanus toxoid, etc. (e.g. via additional Cys-coupling groups at the N-terminus or at the C-terminus).

Preferably the HPV E7 antigen preparation according to the present invention is in liquid form, preferably containing a buffer, especially a phosphate, carbonate or TRIS buffer.

According to a preferred embodiment, the present antigen is therefore provided in a pharmaceutically acceptable form, e.g. further comprising a pharmaceutically acceptable carrier, preferably in a sterile pharmaceutical dosage form, especially as a vaccine.

The HPV E7 antigen preparation may be provided in a pharmaceutically suitable pure form, e.g. wherein the preparation contains more than 40 % HPV protein E7 per total protein, preferably more than 70 % HPV protein E7 per total protein, especially more than 90 % HPV protein E7 per total protein (of course, if no other proteins are used as part of the formulation (e.g. KLH as carrier or albumin as stabiliser)).

A preferred HPV E7 antigen preparation according to the present invention is obtained by a method disclosed herein (as described above).

Preferably, the HPV E7 antigen preparation contains added carbohydrates, especially sugars like maltose, dextrose, glucose, saccharose, mannose or trehalose.

According to another important aspect, the present invention relates to methods for the generation of antibodies specifically recognising the novel antigen provided by the present invention. Therefore, the invention relates to a method for the generation of antibodies against HPV E7 antigen characterised by the following steps:
- immunising mice, rats or rabbits, especially mice, with a HPV E7 antigen preparation according to the present invention,
- producing hybridomas from the immunised mice, and
- screening the monoclonal antibodies produced by the hybridomas for binding ability to the HPV E7 antigen preparation according to the present invention and selecting those hybridomas that produce monoclonal antibodies having a binding ability to the HPV E7 antigen preparation according to the present invention, and
- generating antibodies against HPV E7 antigen from the selected hybridomas.

A further method for the generation of antibodies against HPV E7 antigen is characterised by the following steps:
- contacting a HPV E7 antigen preparation according to the present invention with an antibody phage display or an antibody yeast display library in a soluble screening assay,
- selecting those displayed antibodies that have a binding ability to the HPV E7 antigen preparation according to the present invention of 10⁻⁸ or less, and
- isolating and optionally further expressing the antibodies that have a binding ability to the HPV E7 antigen preparation according to the present invention in a suitable expression system.

Preferably, the generated antibodies are purified and optionally finalised to a diagnostical or pharmaceutical preparation. The antibodies may be provided as IgM or IgG antibodies, especially IgG antibodies.

Polyclonal antibodies according to the present invention can be generated by a method which is characterised by the following steps:
- eliciting an in-vivo humoral response in a non-human animal, preferably a mammal, especially a mouse, a rat or a rabbit against a HPV E7 antigen preparation according to the present invention,
- purifying, especially affinity-purifying, antibodies obtained in the eliciting step that bind to a HPV E7 antigen preparation according to the present invention.

The antibodies provided according to the present invention specifically recognise the HPV E7 antigen preparation according to the present invention. The antibodies specifically detect the "phosphorylated" conformation of E7. Preferred antibodies according to the present invention interact with a large portion of the E7 molecule, e.g. including at least two, preferably at least three, more preferred at least four, especially all of the following amino acids of HPV 16 E7: D4, D14, D21, E18, Y25, E10, and N29.

The present antibodies have preferably an antigen-binding region that is specific for at least two, especially at least three HPV E7 subtypes. Preferred antibodies interact with the Ser phosphorylated region of protein E7. The antibodies according to the present invention produce specific staining in IHC (immune histochemistry) from those mucosal specimens expressing E7, produce specific staining of E7 in immune cytochemistry (ICC) from those mucosal specimens expressing E7, produce specific staining in fluorimetric analysis from those mucosal specimens expressing E7, and possesses a specific affinity for E7 (Kd) of 5nM or below as determined by BIACORE or SET. Due to the specificity of the antibodies according to the present invention, it is possible to provide preferred antibodies with the present invention which do not recognise the zinc finger portion of the E7 protein which may be an advantage in routine testing. With the antibodies according to the present invention, nuclear localized E7 can be detected in a significantly more efficient form than with the antibodies according to the prior art.

Preferably, the antibody according to the present invention is a monoclonal antibody.

Preferably, the antibody according to the present invention is an IgM or IgG antibody, especially an IgG antibody.

The antibody according to the present invention may be any antibody form or antibody fragment which comprises the antigen binding capabilities necessary for specific binding to the novel E7 antigen according to the present invention. For example, the antibody according to the present invention may be a murine, a humanised or a human antibody; a full length antibody, a single chain Fv fragment, a single chain Fv2 fragment, a minibody, a bispecific antibody, a diabody, a triabody, a tetrabody, a di-diabody, a bispecific minibody, a tribiminibody, a Camelid antibody, a Shark antibody, a bispecific single chain Fv fragment, a Fab₂, or a Fab₃. All these forms can be made by a person skilled in the art (see e.g. Holliger et al., Nat. Biotechnol. 23 (2005), 1126-1136 as one example of many); the various antibody scaffolds available can be used to provide the desired format for the present antibodies. Preferred forms are of course those directly obtained by a method as disclosed herein; however, these directly obtained antibodies may also be further processed into any suitable antibody format available to a person skilled in the art.
Preferably, the antibody according to the present invention has a specific affinity K_{D} for a HPV E7 an immobilized or in solution antigen preparation according to the present invention of 10⁻⁷ or less, preferably of 10⁻⁸ or less, especially of 10⁻⁹ or less.
Preferred antibodies according to the present invention are disclosed in the example section. From these experiments, it also follows that an antibody which comprises an amino acid sequence according to SEQ.ID.NO.1 to 10 is a preferred embodiment according to the present invention. Preferred antibodies comprise a CDR3 region with an amino acid sequence according to SEQ.ID.NO.11 to 20.
For specific uses, it is preferred that the antibody according to the present invention is an HPV protein E7 binding antibody fragment, preferably a Fab, Fv, scFv, F(ab')₂ and Fd fragment. Other preferred forms are a chimeric antibody, a humanised antibody or a single-chain antibody.
Preferably, the antibody according to the present invention is conjugated, preferably covalently conjugated, with a label, preferably a radioisotope, an enzyme which generates a detectable product, a fluorescent protein, biotin or avidin; or with a solid support, preferably polystyrene plates or beads and glass slides or beads.
The antibody according to the present invention may be conjugated, preferably covalently conjugated, with a small molecule anti-cancer drug, especially natural and synthetic tubulysins, natural and synthetic epithelons, antracyclins, auristatins or mesothelins.
The antibodies according to the present invention are able to detect the HPV protein E7 in the nucleus of human cells and therefore to specifically recognise active HPV protein E7 during cell proliferation in tissue specimen of HPV infected tissue.
The antibody according to the present invention can be designed to recognise more strains of HPV, preferably the antibody specifically recognises a HPV 16 E7 antigen preparation according to the present invention.

Preferably, the antibody is an IgG antibody.

The antibody according to the present invention may be provided in the form of a diagnostical or pharmaceutical preparation; such a preparation therefore comprises the antibody and a diagnostically or pharmaceutically acceptable carrier, diluent, buffer or storage solution, preferably in a sterile or sterilised state.

According to another aspect, the present invention includes a method for in vitro detecting HPV protein E7 in a biological sample, wherein an antibody according to the present invention is used for binding protein E7 in the sample.

The biological sample may be any biological sample, where E7 is present or where E7 could potentially be present or where there is a risk or a suspicion that E7 could potentially be present (or any biological sample for which presence of E7 should be excluded). Preferred samples are a Pap-smear, a cervical scrape, a cervical lavage, a cervical (carcinoma) biopsy, a mucosal specimen, an anogenital biopsy, a mamma biopsy, a head-or neck biopsy, a bladder biopsy, a colon cancer biopsy, a lung cancer biopsy or a prostate biopsy; a human blood, serum or plasma sample, human saliva or human urine.

For example, such a method may comprise the following steps:
- incubating a biological sample with an antibody according to the present invention and
- measuring and/or detecting specific binding of the antibody to HPV protein E7 in the biological sample.

Such methods can e.g. be used for diagnosing a disease, preferably for diagnosing a sexually transmittable disease, a HPV16-infection cervical cancer, breast cancer/mamma cancer, prostate cancer, bladder cancer, colon cancer, lung cancer, head and neck cancer, penil cancer and/or anogenital cancer/neoplasia (AIN).

The method according to the present invention may comprise the following steps:
- incubating the sample with a monoclonal antibody according to the present invention,
- contacting the monoclonal antibody that is bound to the HPV protein E7 with a second antibody that binds to the monoclonal antibody; wherein the second antibody is conjugated to a detectable compound and
- detecting binding of the second antibody, thereby detecting the HPV protein E7.

Again, the method is suitable for all HPV strains, especially for those which are clinically relevant. Preferably, the HPV protein E7 to be detected is HPV 16 or 18 protein E7, especially HPV 16.

Suitable method formats for performing the method according to the present invention can be selected from immune histochemistry (IHC), immune cytochemistry (ICC), cytofluorimetry (CF), immunofluorescence (IF), enzyme-linked immunosorbent assay (ELISA), Western blot (WB) or dip-stick testing.

Preferably, the method comprises an automatic staining and/or automatic read-out step.

The method according to the present invention can be combined with any further established method relevant in this connection, e.g. it can be combined with the testing of another marker for HPV and/or a tumour.

According to another aspect, the present invention includes a kit for in vitro detecting HPV protein E7 in a biological sample, comprising an antibody according to the present invention.

Preferably, contact of the biological sample with the antibody is performed in a device suitable for providing the sample.

The kit according to the present invention preferably further comprises a second antibody for detecting the antibody according to the present invention. Preferably, this second antibody comprises a label, especially a fluorescent, chromogenic, magnetic or radioactive label.

The kit according to the present invention further comprises reagents for detecting the antibody according to the present invention by an enzyme-linked immunosorbent assay.

The antibody according to the present invention may be provided in an immobilised form in the kit.

In preferred embodiments of the present kit, standard samples for HPV protein E7 may be included, especially also positive and/or negative samples.

The kit according to the present invention is preferably an immune histochemistry (IHC) kit, an immune cytochemistry (ICC) kit, a cytofluorimetry (CF) kit, immunofluorescence (IF), enzyme-linked immunosorbent assay (ELISA), Western blot (WB) or a dip-stick testing kit. Preferably, the kit further comprises means for automatic staining and/or automatic read-out.

The invention is further described by the following examples and the drawing figures, yet without being restricted thereto.

The figures show:
Figure 1A: E. coli expression vector for the production of recombinant E7 proteins,
Figure 1B: provides an example of the essential sequence details for the HPV16 E7 fusion-construct,
Figure 2: Chromatograms and purification profiles of recombinant expressed E7 proteins from various HPV types prior and after post-expression modification,
Figure 2A: Purification profile of the recombinant 16E7 protein purified on an anion exchange resin,
Figure 2B: Purification profile of the recombinant 16E7 protein purified on an anion exchange resin after CKII phosphorylation, Arrow indicates the phosphorylated fraction in the flow through fraction,
Figure 2C: Purification profile of the recombinant 16E7 protein purified on an anion exchange resin after repeated CKII phosphorylation of the peak bound to the anion exchange resin in Figure B obtained after the first CKII phosphorylation, No significant peak in the flow through fraction,
Figure 2D: Profile of the E7 phosphorylated protein in the flow through fraction Figure 2B arrow after three years at 8 to 10°C. Only minor refolding observed,
Figure 2E: Purification profile of the recombinant 18E7 protein purified on an anion exchange resin,
Figure 2F: Purification profile of the recombinant 18E7 protein purified on an anion exchange resin after CKII phosphorylation, Arrow indicates the phosphorylated fraction in the flow through fraction,
Figure 2G: Purification profile of the recombinant 31E7 protein purified on an anion exchange resin,
Figure 2H: Purification profile of the recombinant 31E7 protein purified on an anion exchange resin after CKII phosphorylation, Arrow indicates the phosphorylated fraction in the flow through fraction,
Figure 2I: Purification profile of the recombinant 33E7 protein purified on an anion exchange resin,
Figure 2J: Purification profile of the recombinant 33E7 protein purified on an anion exchange resin after CKII phosphorylation, arrow indicates the phosphorylated fraction in the flow through fraction,
Figure 2K: Purification profile of the recombinant 45E7 protein purified on an anion exchange resin,
Figure 2L: Purification profile of the recombinant 45E7 protein purified on an anion exchange resin after CKII phosphorylation, Arrow indicates the phosphorylated fraction in the flow through fraction, Figure 2 K, L: provides chromatograms from extracts from recombinant His-TEV-HPV 45E7 expression in E.coli with purification profiles prior and after post-expression modification,
Figure 2 M, N, O, P: provides an example of E7 proteins separated in SDS gels with Coomassie staining before (1) and after (2) modification. In M an HPV16E7 preparation is shown. In O an HPV31E7 preparation is shown. In P an HPV45E7 preparation is shown. (X) indicates the molecular mass lane used for size estimation given by the numbers to the left of each gel which measures the migration of proteins with the corresponding size in Kilo Dalton,
Figure 3 and 4: Variable heavy chain (Vh), Variable light chain (Vl) amino acid sequences (Figure 3) and the corresponding CDR3 amino acid sequences of (Figure 4) monoclonal antibodies obtained by the invention are listed. Single amino acid code is shown,
Figure 3A provides the amino acid sequences of HPV16E7 IgG2a antibody ACAB161 variable heavy chain (Vh),
Figure 4A provides the amino acid sequences of the CDR3 amino acid sequence of the ACAB161 variable heavy chain (Vh),

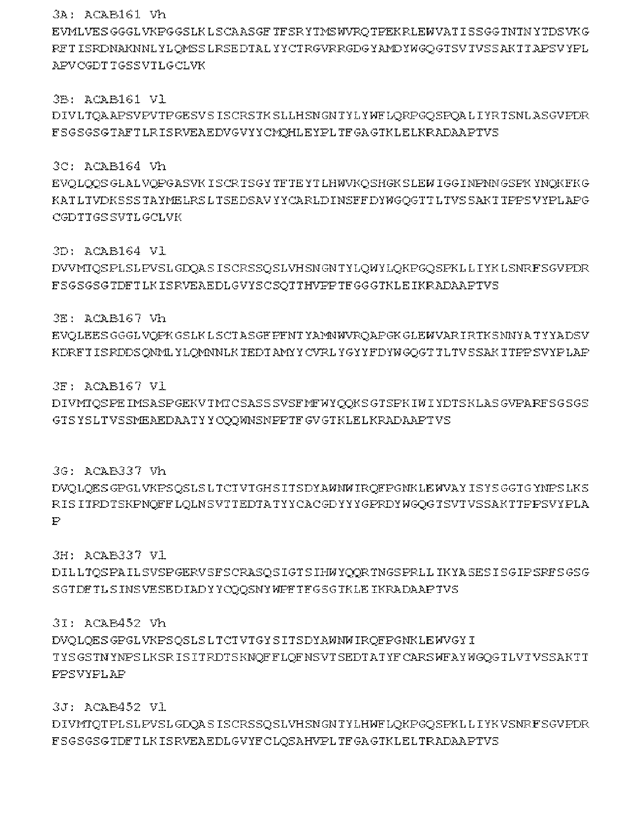

Figure 3B provides the amino acid sequences of HPV16E7 IgG2a antibody ACAB161 variable light chain (Vl),

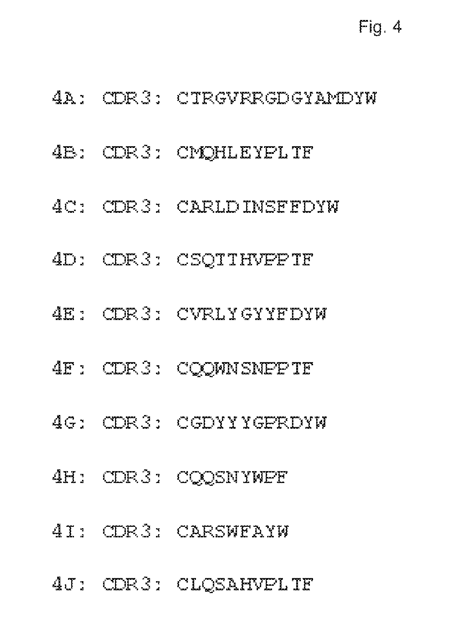

Figure 4B provides the amino acid sequences of the CDR3 amino acid sequence of the ACAB161 variable light chain (Vl),
Figure 3C provides the amino acid sequences of HPV16E7 IgG2a antibody ACAB164 variable heavy chain (Vh),
Figure 4C provides the amino acid sequences of the CDR3 amino acid sequence of the ACAB164 variable heavy chain (Vh),
Figure 3D provides the amino acid sequences of HPV16E7 IgG2a antibody ACAB164 variable light chain (Vl),
Figure 4D provides the amino acid sequences of the CDR3 amino acid sequence of the ACAB164 variable light chain (Vl),
Figure 3E provides the amino acid sequences of HPV16E7 IgG2b antibody ACAB167 variable heavy chain (Vh),
Figure 4E provides the amino acid sequences of the CDR3 amino acid sequence of the ACAB167 variable heavy chain (Vh),
Figure 3F provides the amino acid sequences of HPV16E7 IgG2b antibody ACAB167 variable light chain (Vl),
Figure 4F provides the amino acid sequences of the CDR3 amino acid sequence of the ACAB167 variable light chain (Vl),
Figure 3G provides the amino acid sequences of HPV33E7 IgG1 antibody ACAB337 variable heavy chain (Vh),
Figure 4G provides the amino acid sequences of the CDR3 amino acid sequence of the ACAB337 variable heavy chain (Vh),
Figure 3H provides the amino acid sequences of HPV33E7 IgG1 antibody ACAB337 variable light chain (Vl),
Figure 4H provides the amino acid sequences of the CDR3 amino acid sequence of the ACAB337 variable light chain (Vl),
Figure 3I provides the amino acid sequences of HPV45E7 IgG1 antibody ACAB452 variable heavy chain (Vh),
Figure 4I provides the amino acid sequences of the CDR3 amino acid sequence of the ACAB452 variable heavy chain (Vh),
Figure 3J provides the amino acid sequences of HPV45E7 IgG1 antibody ACAB452 variable light chain (Vl),
Figure 4J provides the amino acid sequences of the CDR3 amino acid sequence of the ACAB452 variable light chain (Vl),

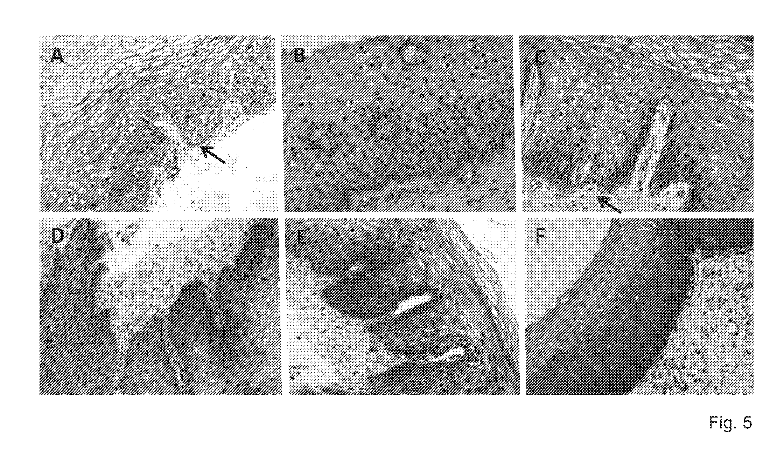

Figure 5: Immunohistochemistry (IHC) staining of various human biopsies,
Figure 5A: Immunohistochemistry (IHC) staining of human biopsies in regression as indicated by arrow pointing to negative basal cells using ACAB161,
Figure 5B: Immunohistochemistry (IHC) staining of human biopsies in a early stage CIN1/2 using ACAB161,
Figure 5C: Immunohistochemistry (IHC) staining of human biopsies in CIN2/CIN3 in regression as indicated by arrow pointing to negative basal cells using ACAB161,
Figure 5D: Immunohistochemistry (IHC) staining of human biopsies in CIN2/CIN3 in advanced progression using ACAB452,
Figure 5E: Immunohistochemistry (IHC) staining of human biopsies in CIN2/CIN3 in advanced progression using ACAB161,
Figure 5F: Immunohistochemistry (IHC) from a historical sample of more than 30 years of age staining of human biopsies in CIN2/CIN3 in advanced progression using ACAB161,

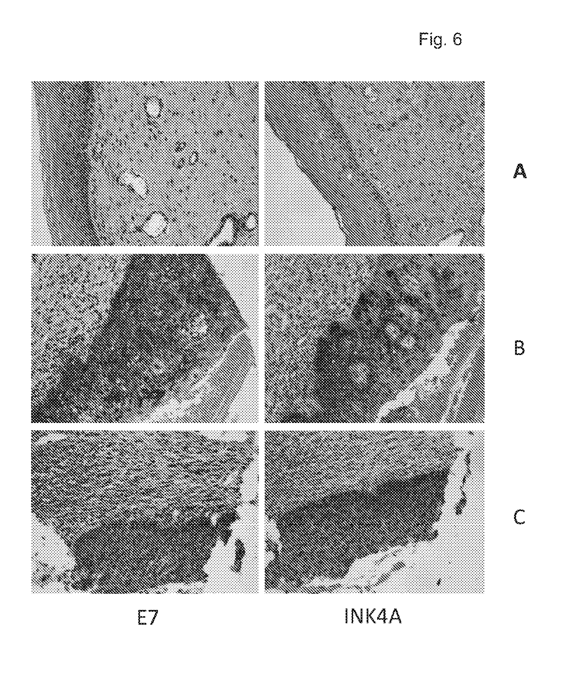

Figure 6: Immunocytochemistry of HPVE7 (E7, right row)used for staging of cervical cancer biopsies in comparison to the p16INK4A (INK4, left row) marker. Whereas E7 Shows early stage detection in CIN1 (A) up to late stage (B, C), as compared for p16INK4A with no detection in CIN1 (A), different pattern in CIN2/3 (B) and identical pattern in CIN3 (C). Combination of markers can analyse progression and distinguish borderline cases were CIN1 or CIN1/2 is not clearly identified,

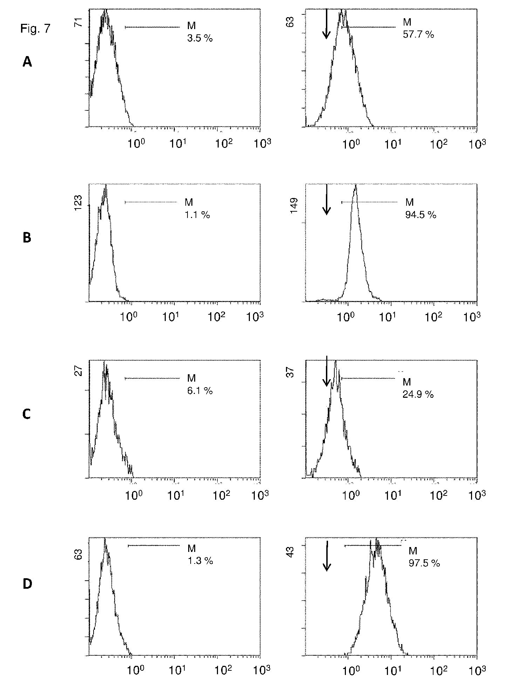

Figure 7: Cytofluorimetric analysis with the mAbs of the invention in using different cervical cancer cell isolates, left panel fluorescence of secondary antibody alone, right panel HPVE7 specific signal, arrows on right panel indicate position of secondary antibody background, histograms are shown X-axis shows fluorescence in logarithmic scale (FL1 488 excitation laser), Y axis events analysed (at least 10.000 cells were analysed/sample),
Figure 7A shows the HPV39E7 containing ME180 cells,
Figure 7B shows the HPV45E7 containing M576 cells,
Figure 7C shows the HPV16E7 containing Siha cells, tumour cells that contain a single copy of HPV16E7 DNA,
Figure 7D shows the HPV16E7 containing Caski cells, tumour cells that contain more than 100 copies of HPV16E7 DNA,

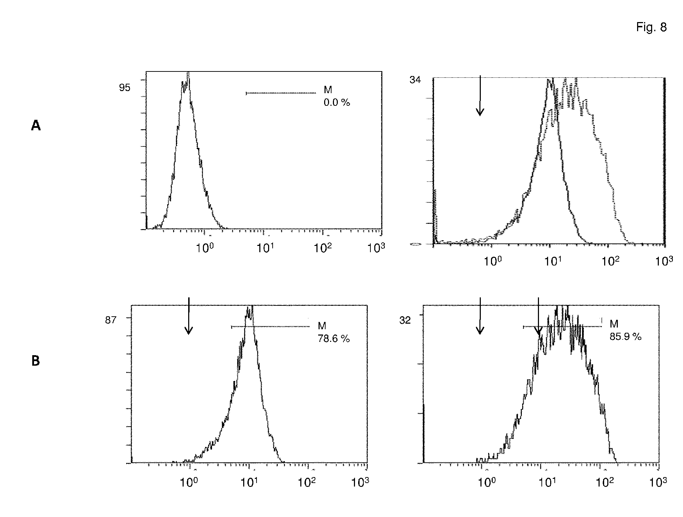

Figure 8 shows the nuclear distribution of HPV16E7 containing Caski cells,
Figure 8A left panel shows the fluorescence of secondary antibody alone, right panel overlay of histograms separately shown in B total cell stain (full line-more left), and nuclear staining of E7 (dotted line-more right), arrow indicates fluorescence intensity of secondary antibody alone,
Figure 8B left panel shows the fluorescence of total cell stain for E7, arrow indicates fluorescence of secondary antibody alone, and right panel shows nuclear staining of E7; arrow indicates fluorescence of secondary antibody and total cell stain (arrow more right),

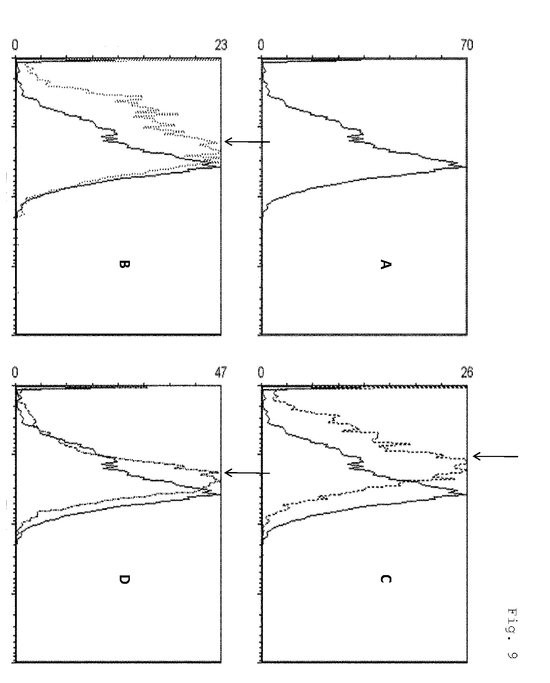

Figure 9 shows the cell cycle distribution of E7 in a cytofluorimetric assay. Histograms are shown, X-axis shows fluorescence in logarithmic scale (FL1 488 excitation laser), Y axis events analysed (at least 10.000 cells were analysed/sample), Figure 9A shows E7 analysis after serum-starvation,
Figure 9B shows E7 analysis 1 hour after serum release (solid line) arrow indicates serum starvation level in (A, dotted line),
Figure 9C shows E7 analysis 6 hours after serum release (solid line) arrow indicates serum starvation level in (A, dotted line),
Figure 9D shows overlay of 1hour (dotted line and arrow) and 6 hour serum release (solid line),

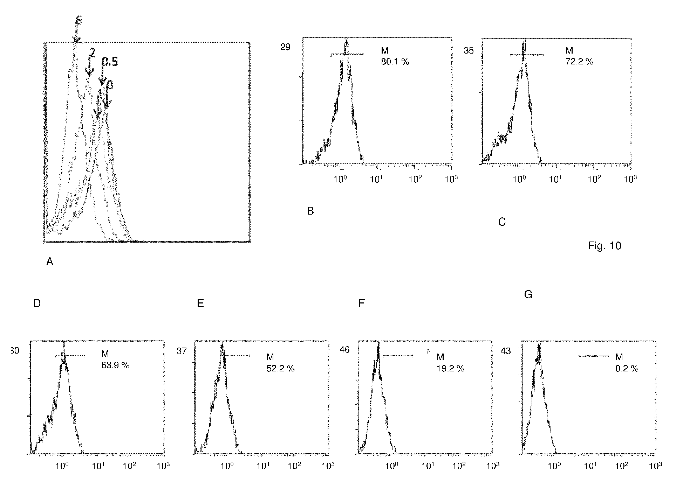

Figure 10: Cytofluorimetric analysis of E7 with the mAbs of the invention using cervical cancer cell isolates Caski and E7 specific staining after different timepoints of exposure to Cycloheximide (Cyh), (A) overlay blot is shown of cells incubated with Cyh for 0.5 hours (arrow with 0.5), for 1 hour (arrow with 1), for 2 hours (arrow with 2), for 6 hours (arrow with 6),and no Cyh (arrow with 0); Panel B shows auto-fluorescence of secondary antibody alone, panel C, D, E, F, G, shows the individual blots as described for the overlay in A representing timepoints of Cyh incubaton of 0, 0.5, 1, 2, and 6 hours respectively. Histograms are shown, X-axis shows fluorescence in logarithmic scale (FL1 488 excitation laser), Y axis events analysed (at least 10.000 cells were analysed/sample),

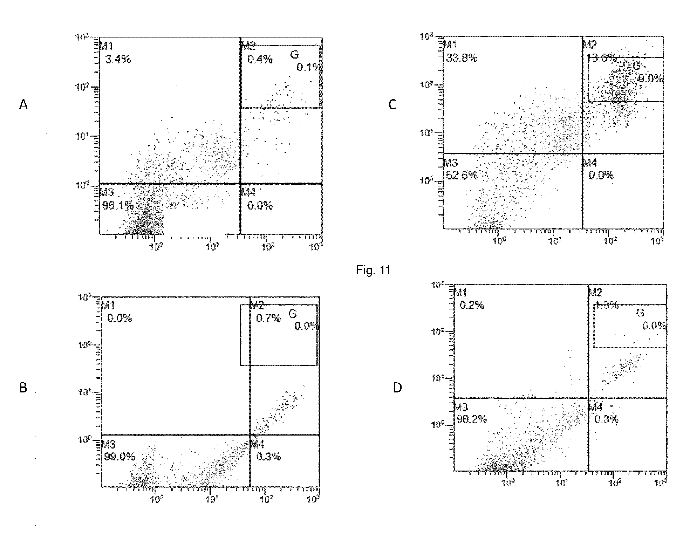

Figure 11: Gynecological liquid pap smear samples analysed with E7 specific staining in cytofluorimetry analysis as a single factor read out. Dot blots are shown, X-axis shows sideward scattering and Y axis fluorescence in logarithmic scale (FL1 488 excitation laser), at least 20.000 cells were analysed/sample. Dot blots were separated into four regions and E7 positive cells are detected in the upper left region,
Figure 11A shows E7 analysis of a sample from a HPV58 DNA positive individual using ACAB161 antibody,
Figure 11B shows E7 analysis of a sample from a HPV16 DNA positive individual using ACAB161 antibody,
Figure 11C shows E7 analysis of a sample from a HPV-DNA negative individual using ACAB161 antibody,
Figure 11D shows E7 analysis of a sample from a HPV-DNA negative individual using ACAB161 antibody,

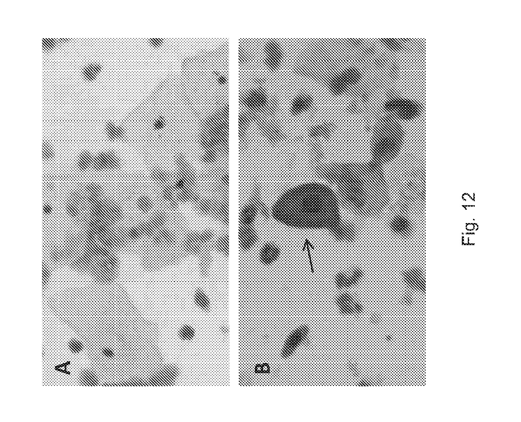

Figure 12: Cytochemical analysis of E7 in gynecological samples using the antibodies of the invention. Liquid Pap samples are used and cells are fixed on microscopic slides. Pictures show E7 positive staining of positive HSIL sample (arrow in B) and negative staining for E7 in negative PAP sample (A), counterstain with Hematoxylin.

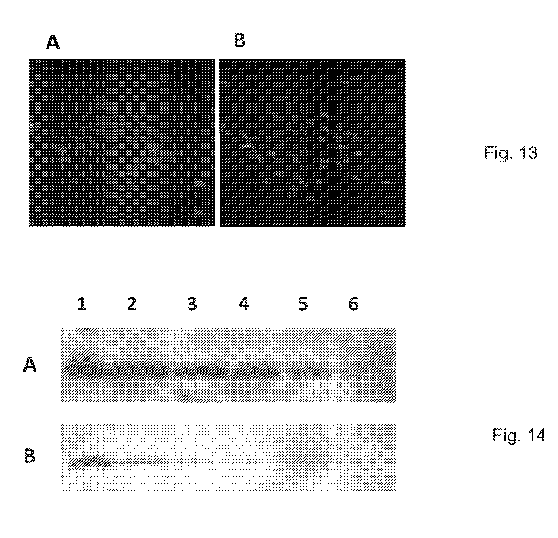

Figure 13: Immunofluorescence analysis of Caski cells using E7 specific detection. E7 staining using a secondary antibody coupled with FITC for detection (A) and staining for DNA (B, DAPI) image from the same field.
Figure 14: Western-blot of high (A, Caski) and low copy number cancer cells (B, Siha). For detection E7 specific antibodies and ICL development were used. Amount of protein extract loading per lane 1 was equivalent 55.000 cells, lane 2, 25.000 cells, lane 3, 12.500 cells, lane 4, 6250 cells, lane 5, 3000 cells, and lane 6, 1500 cells,

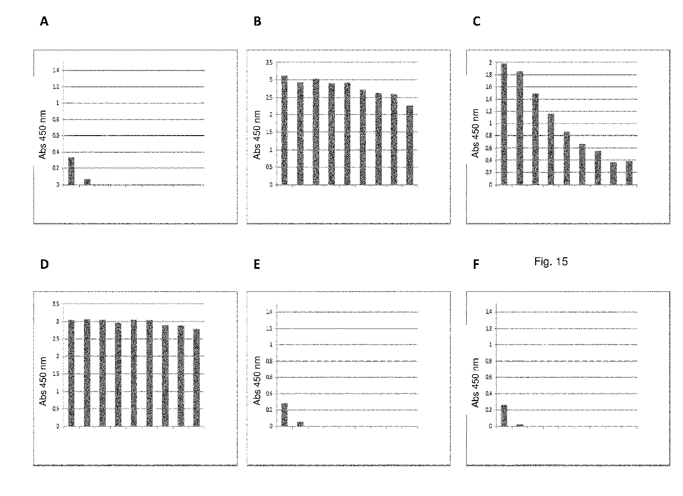

Figure 15: ELISA analysis using ACAB452 antibodies and the various antigens immobilised to polystyrene plates. Bars represent intensity of signal at 450nm shown on X axis, Antigens starting from 800 nM diluted down to 0.1 nM from the left to the right in steps of 3 times dilution. Antibody used ACAB452. E7 antigens were HPV16E7 (A), HPV18E7 (B), HPV31E7 (C), HPV45E7 (D), HPV52E7 E, HPV58E7 (F),

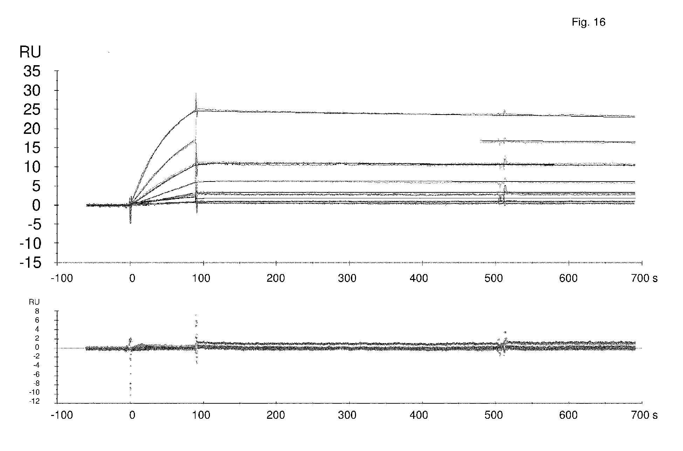

Figure 16: provides exemplary Biacore kₒₙ and k_{off} curve fits for ACAB164. Y-axis Response Units (RU), X axis timepoints in seconds from 0 to 700 seconds shown. Lower panel shows the "residuals" of the measurements.

### EXAMPLES

### EXAMPLE 1: Expression Vectors for a Recombinant HPV E7 protein expression in E. coli

A special vector was used for the expression and generation of different recombinant alpha or beta-papillomavirus E7 proteins in *E coli.* This vector contained an IPTG inducible promoter for E7 expression. The vector allows for the production of fusion proteins with low to moderate expression levels. The vector contains an N-terminal 6xHistidine tag followed by a recombinant TEV protease cleavage site followed by a site for proper E7 reading frame insertion. All the DNA constructs were done by recombinant techniques well known in the art and all E7 containing DNAs were synthesized in vitro (Gene Art). In part codon optimized E7 DNA for E.coli expression was used. The following entire reading frames from the Alpha-papillomavirus Types and three entire reading frames from the Beta-papillomavirus were constructed, for the expression of E7 as 6xHistidine and TEV cleavage site containing fusion-proteins. The amino acids of the expressed E7 proteins from the various HPV types were according to the Expasy ViralZone database. The sequences used have the following Sequence Numbers and Identifiers (in parenthesis)

Amino acid sequences for Alpha-papillomavirus were P03129 (VE7_HPV16); P04020 (VE7_HPV11); P06788 (VE7_HPV18); P17387 (VE7_HPV31); P06429 (VE7_HPV33); P27230 (VE7_HPV35); P24837 (VE7_HPV39); P36829 (VE7_HPV40); P27231 (VE7_HPV42); Q705H9 (Q705H9_HPV43); Q80914 (VE7_HPV44); P21736 (VE7_HPV45); P26558 (VE7_HPV51); P36831 (VE7_HPV52); P36832 (VE7_HPV53); Q81019 (VE7_HPV54); P36833 (VE7_HPV56); P26557 (VE7_HPV58); Q81965 (Q81965_HPV59), Q80956 (VE7_HPV66); P54668 (VE7_HPV68); P50785 (VE7_HPV70); Q9IR58 (Q9IR58_HPV82); Q84292 (VE7_HPV6A);

Amino acid sequences for Beta-papillomavirus were: P06932 (VE7_HPV05); P06430 (VE7_HPV08); Q80908 (VE7_HPV38);

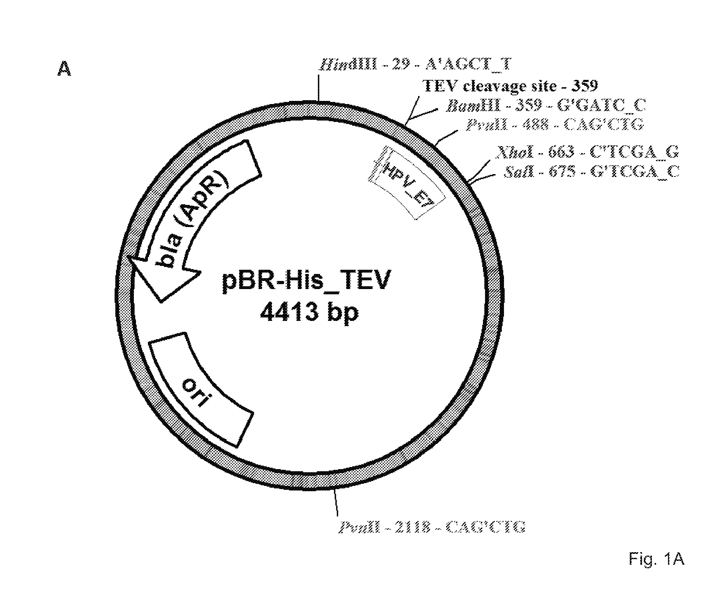

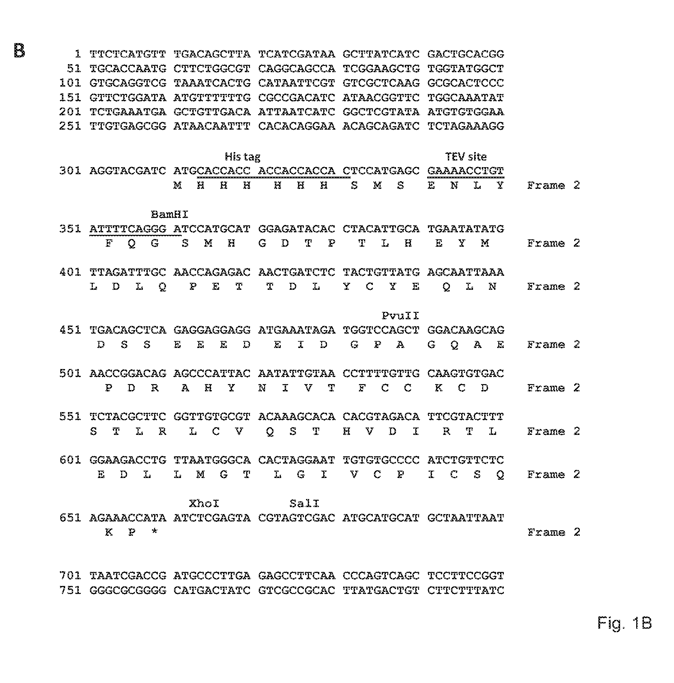

The vector is shown in Figure 1A and one example of the essential sequence details for the HPV16 E7 fusion-construct in Figure 1B.

### EXAMPLE 2: HPV-E7 protein purification with novel folding properties

For the generation of E7 as a suitable antigen for immunization a new procedure was developed that resulted in a unique new E7 preparation with highly un-expected features. Proteins were obtained showing a robust stable folding at 8 to 15°C for more than 3 years. For this an important step was invented to produce stable folded E7 proteins and separate them by chromatographic purification from relaxed partly unfolded structures. These stable folded protein fractions were used for the generation of monoclonal antibodies with new not described features.

### A Recombinant production of E7

The E7 protein expression was obtained by plasmid transformation using the low expression level vector pBR-His-TEV-E7 containing the various DNA coding for high risk HPVE7 open reading frames as described in EXAMPLE 1. The E.coli host XL1 BLUE obtained as chemically competent cells (Stratagene) were used for plasmid transformation. The mixture was plated on LB plates containing Ampicillin (75 mg/l) and incubated overnight at 37°C. Following overnight incubation of the cells on the plate, 4 ml of LB was added to the plates and colonies were scraped into the liquid. The liquid from one bacterial plate was used to inoculate a 400 ml liquid culture medium of LB Ampicillin (75mg/l) and ZnCl₂ was added to the liquid culture to a final concentration of 0.1 mM. This inoculation step proofed fundamental as E7 proteins expressed with suitable expression vectors tend to lose the plasmid due to the toxic nature of the protein in *E coli.* The toxicity was found to be a sign for proper expression and allows for the required folding that can be purified in a properly developed downstream process shown in this invention. It was observed that high level including inclusion body expressing systems that do not show toxicity only rarely bring about the proteins of the invention. The culture was grown to OD₆₀₀ of 0.6 and protein expression from a lac Z regulated promoter was induced by adding IPTG at a final concentration of 0.2 mM for 2 hrs at 30°C. Cells were collected by centrifugation at at 4000xg for 15 minutes at 8-10°C. The pellet was washed once with buffer 1 (Table 1, for buffers). Pellets were either stored at -80 °C or further processed directly. Pellets were resuspended in 4 ml/g of buffer 1 (containing COMPLETE Protease Inhibitor Cocktail; Roche # 05056489001) and vortexed to resuspend cell aggregates. Homogenization was done by 3 cycles @ 1000 bar, with temperature kept between 8-10°C using a GEA NS 1001L-2K homogenizer (GEA Niro Soavi, via Da Erba Edoardi 29/A, 43100 Parma, Italy). The homogenate was clarified by centrifugation at 25000 x g for 30 minutes at 8-10°C. Following centrifugation the clarified supernatant was sterile filtered on a Stericup PVDF Durapore^{®} 0.22 µm filtration device (Millipore).

### B Downstream processing the recombinant E7 proteins

The sterile filtered clarified supernatant was purified on a HisTrap 6 FF 5 ml (GE Healthcare # 17-5255-01) affinity column and an automatic purification device, ÄKTA Purifier UPC 10, (GE Healthcare Bio-Sciences AB, Björkgatan 30, 75184 Uppsala, Sweden) was used. The purification was performed at a constant flow of 5 ml/min. The sample was loaded onto the HisTrap column pre-equilibrated with buffer 2 (Table 1). Unbound sample was washed out with 20 column volume (CV) of buffer 2 and the column was then washed with 5 x CV of buffer 3 to decrease the salt concentration and to remove residual contaminants. The protein was eluted with a step gradient using 5 x CV of buffer 4 (Table 1).

The presence of a photolytic cleavage site allows to prepare E7 molecules without the 6 x HIS tag. For this the E7 proteins can be digested after the HisTrap 6 FF purification with TEV protease according to the art. The resulting E7 protein digestion products are then processed in the same way as described for the HIS-TEV-E7 fusion proteins products below. The resulting E7 proteins showed the same behaviour as was the case for the HIS containing proteins, thus demonstrating that the 6 x HIS tag does not influence the behaviour of the post-expression modified E7 proteins.

The quality of the fractions was analyzed by SDS-polyacrylamide gel electrophoresis, using a 15% acrylamide/bisacrylamide gel. Fractions from the elution step were pooled and dialyzed for 16 hrs at RT against a 20 X volume excess of buffer 5 (Table 1). Dialysis was done in a Spectrapore Membrane 3500 MWCO (Spectrum Labs # 132-720). The dialyzed sample was centrifuged at 25000 x g for 30 minutes at 8-10°C.

The supernatant was further purified with the Anion Exchange resin HiTrap Q Sepharose FF 1 ml (GE Healthcare # 17-5053-01) with an automatic purification device, ÄKTA Purifier UPC 10, (GE Healthcare Bio-Sciences AB, Björkgatan 30, 75184 Uppsala, Sweden). Samples were loaded onto the buffer 5 (Table 1) pre-equilibrated HiTrap Q column. The purification was performed at a constant flow of 0.5 ml/min. Flow-through was collected. Unbound sample was washed out with 10 column volume (CV) of buffer 5. The protein was eluted with a 5 x CV linear gradient from 0 to 100% CV buffer 6 (Table 1). At this step all the different E7 recombinant proteins elute at around 450 mM NaCl. This fraction was dialyzed against a 50 X volume excess of buffer 7 for 16 hours at RT using a Spectrapore Membrane 3500 MWCO (Spectrum Labs # 132-720).

The quality of the fractions was analyzed by SDS-polyacrylamide gel electrophoresis, using a 15% acrylamide/bisacrylamide gel. Fractions from the elution step were pooled and dialyzed for 16 hrs at RT against a 20 X volume excess of buffer 7 (Table 1). Dialysis was done in a Spectrapore Membrane 3500 MWCO (Spectrum Labs # 132-720). The dialyzed sample was centrifuged at 25000 x g for 30 minutes at 8-10°C.

### C Post-expression modification of E7 recombinant proteins

E7 is described in the literature as a phosphoprotein and is phosphorylated by Casein Kinase II (CKII). The phosphorylation consensus site in high risk HPV-E7 is present either as GXSXXX (one phosphor-S) (species 7 39E7, 68E7) SXSXXX (two phosphor-S) by one amino acid (species 7) or a doublet SSXXX (two phosphor-S) (species 5, 6, 9) which represent a CKII consensus site for phosphorylation (X is either E or D).

In the new procedure the recombinant proteins E7 are phosphorylated in a robust procedure using either recombinant Casein Kinase II or Casein Kinase II (CKII) fractions highly purified from cycloheximide starved rat liver or Casein Kinase II containing fractions from calf thymus extracts purified in a downstream process that employs recombinant E7 as assay substrate together with the Casein Kinase II specific GTP as nucleotide source. E7 Proteins obtained with this procedure if phosphorylated are always phosphorylated on all described CKII consensus sites, as was determined by mass spec analysis or by experiments with radioactive GTP labeling. It was seen for example for species 9 that always either both sites are phosphorylated or none. For the generation of CKII phosphorylated E7 proteins the dialyzed E7 protein fraction eluting from the 450 mM fraction of the Anion Exchange HiTrap Q Sepharose was used. Protein concentration for the phosphorylation protocol was 1-0.5 mg/ml. For the CKII kinase reaction the phosphorylation buffer 8 (Table 1) was used. CKII phosphorylation reaction was at 25°C for 4 hours. The reaction products were dialyzed for 16 hrs at RT against a 20 X volume excess of buffer 5 (Table 1). Dialysis was done in a Spectrapore Membrane 3500 MWCO (Spectrum Labs # 132-720). The dialyzed sample was centrifuged at 25000 x g for 30 minutes at 8-10°C. LPS was analysed with the PTS-Endosave System (Charles River) and nucleic acid content with PICO green assay (Invitrogen) was below 10ng/ml.

### D Purification of CKII phosphorylated E7 protein as a robust stable folding fraction

The dialysed CKII reaction mixture was purified with a second Anion Exchange resin HiTrap Q Sepharose FF 1 ml (GE Healthcare # 17-5053-01) with an automatic purification device, ÄKTA Purifier UPC 10, (GE Healthcare Bio-Sciences AB, Björkgatan 30, 75184 Uppsala, Sweden). Samples were loaded onto the buffer 5 (Table 1) pre-equilibrated HiTrap Q column. The purification was performed at a constant flow of 0.5 ml/min. The flow-through resulted in a new highly significant peak that was not obtained on the first Anion Exchange step. This peak was identified as the phosphorylated E7 fraction, highly purified as determined by mass-spectroscopic analysis. This was further confirmed by running the column in the presence of spike E7 protein CKII phosphorylated in the presence of P32 GTP nucleotides which results in radioactive fractions with stochiometrically fully phosphorylated E7 protein in the flow through fraction. After washing with 10 x CV of buffer 5 the residual bound protein was eluted with a 5 x CV linear gradient from 0 to 100% CV buffer 6 (Table 1). An E7 recombinant proteins fraction elutes at around 450 mM NaCl. This fraction was dialyzed against a 50 X volume excess of buffer 7 for 16 hours at RT using a Spectrapore Membrane 3500 MWCO (Spectrum Labs # 132-720).

The separation of the CKII phosphorylated E7 fraction in the low salt flow through fraction of an Anion exchange resin was a highly unexpected finding. Different Anion exchange resins such as MonoQ, DEAE Sepharose all resulted in the same reproducible result, CKII E7 phosphorylated with the method described separates in the 0.1 mM flow through fraction. Given the negative charged nature of all the E7 proteins elution of the non-phosphorylated fraction at 450 mM was not surprising, but the addition of two to four (depending on E7 species) more negative charges introduced by the phosphate groups from the CKII phosphorylation was expected to bind stronger to the resin and is not contained in the flow through fraction at all. One explanation is that due to the phosphorylation event the structure of the E7 proteins is changed and the negative charges are no more surface exposed. In fact CKII kinase sites are described as low turnover sites and thus do not necessarily have to be exposed to the surface. So far attempts to dephosphorylate the CKII phosphorylated E7 molecules of the invention with PP2A and Phosphatase I were not successful indicating difficult to access substrates. Moreover interestingly, the fraction that still binds to the anion exchange column cannot be modified in a second CKII reaction in order to get into the flow through fraction.

**Table 1**

| | Purpose | Composition | pH |
|---|---|---|---|
| Buffer 1 | Bacterial pellet washing buffer | 20 mM Tris, 50 mM NaCl | 8.0 |
| Buffer 2 | Affinity chromatography binding buffer | 50 mM NaH₂PO₄/Na₂HPO₄, 1 M NaCl, 20 mM Imidazole | 8.0 |
| Buffer 3 | Affinity chromatography washing buffer | 20 mM TEA (Tri-Ethanol Amine), 0.1 M NaCl, 20 mM Imidazole | 8.0 |
| Buffer 4 | Affinity chromatography elution buffer | 20 mM TEA, 0.1 M NaCl, 0.5 M Imidazole | 7.5 |
| Buffer 5 | Anion exchange buffer A | 20 mM TEA, 0.1 M NaCl | 7.5 |
| Buffer 6 | Anion exchange buffer B | 20 mM TEA, 1 M NaCl | 7.5 |
| Buffer 7 | Phosphorylation reaction dialysis buffer | 10 mM TEA, 1.2 mM EDTA, 0.1 M NaCl | 7.8 |
| Buffer 8 | Phosphorylation reaction | 1.1 mg/ml E7, 300 µM ATP(or 300µM GTP), 10 mM MgCl₂; CKII Kinase, 10 mM TEA, 1.2 mM EDTA, 0.1 M NaCl | 7.8 |

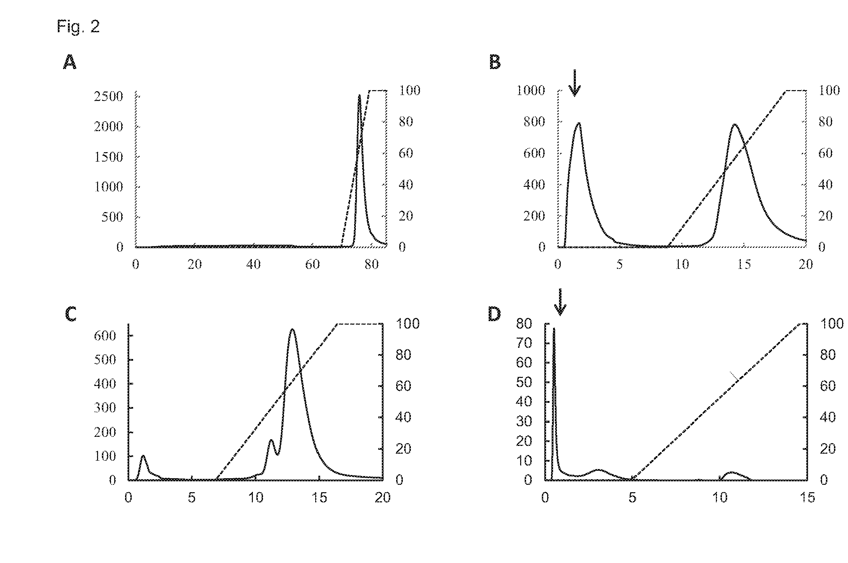

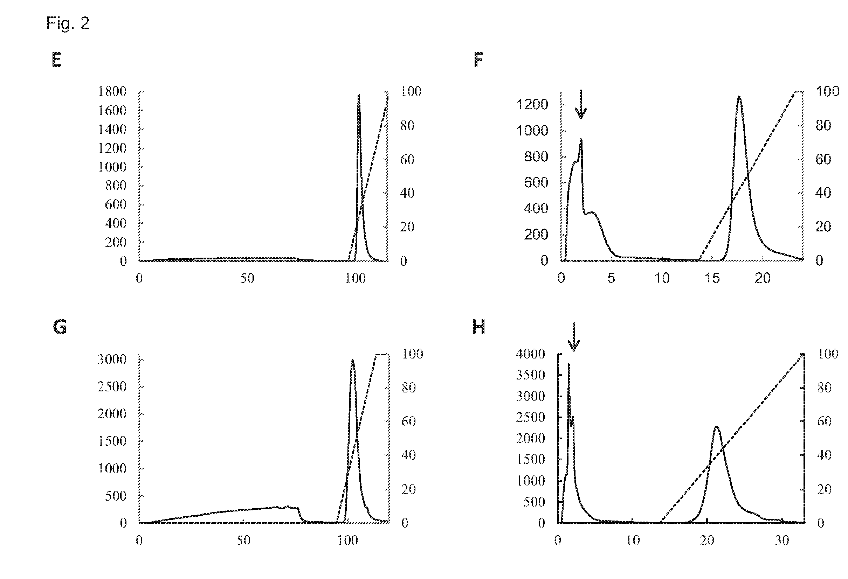

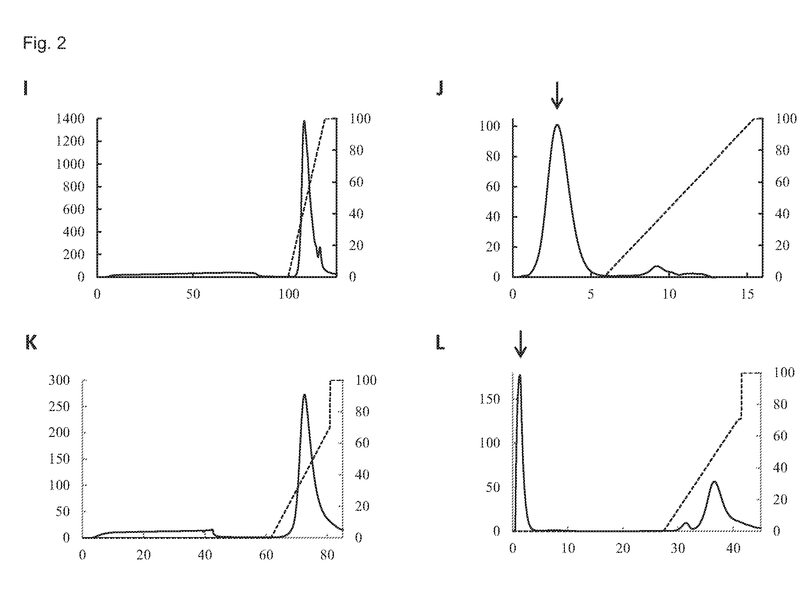

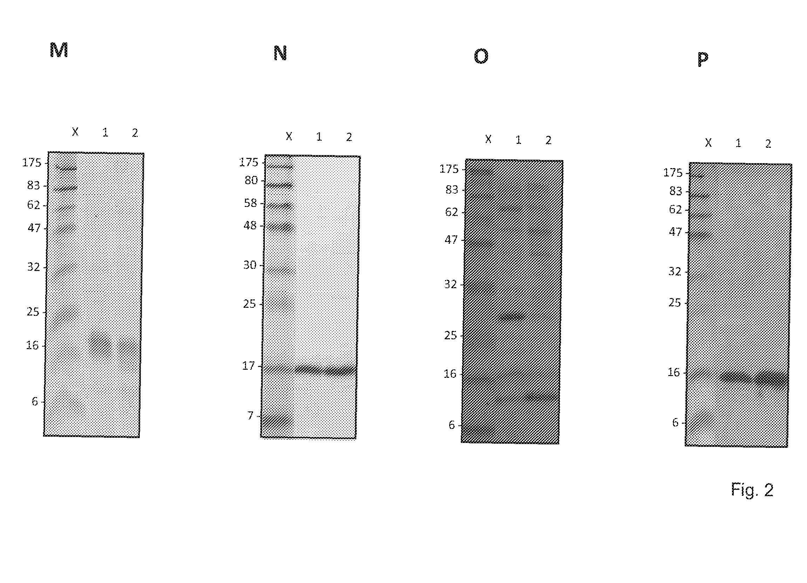

FIGURE 2 Chromatograms and purification profiles of recombinant expressed E7 proteins from various HPV types prior and after post-expression modification.

Figure 2 A Purification profile of the recombinant 16E7 protein purified on an anion exchange resin Figure 2 B Purification profile of the recombinant 16E7 protein purified on an anion exchange resin after CKII phosphorylation, Arrow indicates the phosphorylated fraction in the flow through fraction,

Figure 2 C Purification profile of the recombinant 16E7 protein purified on an anion exchange resin after repeated CKII phosphorylation of the peak bound to the anion exchange resin in

Figure B obtained after the first CKII phosphorylation, No significant peak in the flow through fraction,

Figure 2D Profile of the E7 phosphorylated protein in the flow through fraction Figure 2B arrow after three years at 8 to 10°C. Only minor refolding observed.

Figure 2 E Purification profile of the recombinant 18E7 protein purified on an anion exchange resin

Figure 2 F Purification profile of the recombinant 18E7 protein purified on an anion exchange resin after CKII phosphorylation, Arrow indicates the phosphorylated fraction in the flow through fraction,

Figure 2 G Purification profile of the recombinant 31E7 protein purified on an anion exchange resin

Figure 2 H Purification profile of the recombinant 31E7 protein purified on an anion exchange resin after CKII phosphorylation, Arrow indicates the phosphorylated fraction in the flow through fraction,

Figure 2 I Purification profile of the recombinant 33E7 protein purified on an anion exchange resin

Figure 2 J Purification profile of the recombinant 33E7 protein purified on an anion exchange resin after CKII phosphorylation, Arrow indicates the phosphorylated fraction in the flow through fraction,

Figure 2 K Purification profile of the recombinant 45E7 protein purified on an anion exchange resin

Figure 2 L Purification profile of the recombinant 45E7 protein purified on an anion exchange resin after CKII phosphorylation, Arrow indicates the phosphorylated fraction in the flow through fraction,

### EXAMPLE 3: HPVE7 purification from S. cerevisiae

The protocol for the purification of recombinant HPV E7 from *E coli,* was adapted for the purification of a recombinant HPV E7 protein from the yeast *S. cerevisiae.* For this the E7 spanning reading frame shown in EXAMPLE 1 was cloned into the yeast pGAL vector that allows for expression of proteins under the GAL1 promoter and galactose induction. Extracts were prepared as described in EXAMPLE 1 with slight modifications as follows. Homogenization was done by 3 cycles @ 1500 bar, with temperature kept between 8-10°C using a GEA NS 1001L-2K homogenizer (GEA Niro Soavi, via Da Erba Edoardi 29/A, 43100 Parma, Italy). Following homogenization before loading on the Affinity column, the sterile filtered sample was adjusted to 2M NaCl and DNA was precipitated by stirring on ice in the presence of 20% Poly ethylene glycol 6000. After centrifugation at 25000g for 30 minutes supernatant was proceeded into downstream processing on as in EXAMPLE 2 except that the kinase reaction was done already after the HisTrap 6 FF affinity column. Eukaryotic cells including yeast cells express endogenous CKII kinase and thus phosphorylation takes place *in vivo,* however in the recombinant expression systems phosphorylation was found incomplete and therefore for a complete phosphorylation of both serines of the 16E7 substrate and to obtain the folding as was observed for E.coli produced proteins, the additional in vitro Kinase reaction was introduced. After this step a HiTrap Q Sepharose FF anion exchange column was used for the separation of the phosphorylated and proper folded fraction from the phosphorylated or partial phosphorylated and non-folded fraction that properly binds to the HiTrap Q Sepharose FF already.

### EXAMPLE 4: HPVE7 purification from mammalian HEK293 cell culture

The E7 spanning reading frame shown in EXAMPLE 1 was cloned into the mammalian expression vector pCDNA3 (Stratagene). Extracts were produced and proteins were purified as described for yeast in EXAMPLE 3 except that Extract was homogenized by 2 cycles @ 1500 bar, with temperature kept between 8-10°C using a GEA NS 1001L-2K homogenizer (GEA Niro Soavi, via Da Erba Edoardi 29/A, 43100 Parma, Italy). Downstream process was as in EXAMPLE 3 and E7 protein fractions were isolated from the HiTrap Q Sepharose FF anion exchange column flow through fraction.

### EXAMPLE 5: Generation of monoclonal HPV-E7 antibodies A Immunization of the mice

For each E7 protein, four BALB/c mice were immunized by intraperitoneal injections of purified protein. On day 1, mice were injected with 30 µg purified protein in complete Freund's adjuvant. Injections were repeated on day 21 with 30 µg purified protein in incomplete Freund's adjuvant and on day 42 with 10 - 50 µg purified protein in incomplete Freund's adjuvant. On days 45 and 46, injections of 10 µg purified protein in sterile PBS were administered. Cell fusion was performed on day 47 as below "B Hybridoma Fusion".

### B Hybridoma Fusion

Ten days prior to start of the fusion, mouse myeloma cells (SP-2) were recovered and expanded. For one mouse, 2-3 x 300 ml flasks were used. Booster injections (10-20 µg of antigen in a total volume of 100 µl sterile PBS injected intraperitoneally or into tail vein) were administered 3, 4 and 5 days before the fusion. Feeder cells were prepared one day before the fusion and seeded in 96-well plates to allow mouse macrophages to adhere.

For cell fusion, spleens were removed from mice under sterile conditions and transferred to a petri dish. A syringe was used to make several holes in the spleen into which 5 ml serum-free DMEM was added to release the cells. This step was repeated twice. The spleen was then torn into pieces, washed with 5 ml serum-free DMEM and transferred to a sterile universal tube. The number of splenocytes was estimated and between 100-300 million cells were used for fusion. Sediment was allowed to settle for 2 minutes, supernatant was removed and centrifuged at 350g for 10 minutes at 37°C. The pellet was resuspended in serum-free DMEM up to 20 ml. Once again, sediment was allowed to settle for 2 minutes, supernatant was removed and centrifuged at 350g for 10 minutes at 37°C. During the centrifugation step, myeloma cells were harvested, counted and washed once with serum-free DMEM. The washed splenocytes and myeloma cells were mixed at a ratio of 5:1 (splenocytes:myeloma cells) and centrifuged at 450g for 7 minutes at 37°C and the supernatant was discarded. The pellet was resuspended in the remaining medium and 0.7 ml PEG was added slowly drop by drop while the suspension was gently mixed. PEG 1500 from Merck is recommended. The cell suspension was gently mixed for another minute and 10 ml serum-free DMEM was added slowly drop by drop (at a rate of 1 ml per minute) and then centrifuged at 350g for 5-6 minutes at 37 °C. The supernatant was discarded and the cell pellet was resuspended in 50 ml AH DMEM (containing 1 µg/ml azaserine, 10⁻⁴ M hypoxanthine, 20% fetal bovine serum, 300 mg/l L-glutamine penicillin/streptomycin. This was divided between 9 x 96-well plates, adding 1-2 drops per well. Three days later, one drop of AH DMEM was added to each well. Medium was removed seven days later and replaced with fresh medium. The first hybridoma supernatants were ready for screening on the 11^{th} or 12^{th} day. Hybridoma cells were grown for supernatant containing antibodies, master cell stock and cloning. Cloning was performed with limited dilution and was repeated once to ensure the monoclonality of hybridomas. After cloning, hybridomas were grown in DMEM supplemented with 10% fetal bovine serum, 300 mg/l L-glutamine and penicillin/streptomycin.

### C Screening of hybridoma

For the screening of the hybridoma supernatants nitrocellulose immobilized proteins were used. First screening was done in parallel with either against rabbit anti-mouse Ig or against purified protein bound to nitrocellulose (NC) membrane (100 µg of rabbit anti-mouse Ig/ml of PBS or 20 µg of purified E7 protein/ml of PBS were incubated with the membrane and incubated for 2h at 37°C). The nitrocellulose membrane was blocked with 10% fetal calf serum (FCS) in DMEM for 2 h at room temperature and 2 µl of supernatant from each tested hybridoma was spotted onto the NC membrane. The membrane was incubated for 2 h at room temperature and then washed 3 times for 5 minutes in PBS. The membrane was incubated for 1 h with rabbit anti-mouse Ig labeled with horseradish peroxidase diluted 1:100 in DMEM + 10% FCS and washed 3 times for 5 minutes in PBS. The reaction was developed using 1, 4-chloronaphtol.

Dot blot positive hybridoma clones were further tested in Western blots as described in EXAMPLE 16 below.

### EXAMPLE 5: Sequencing of the obtained mAbs revealed novel CD3 structures in both heavy and light chain context

Amino Acid sequencing was done with the following methods. All sequences were confirmed by reverse transcription and DNA sequencing according to the art. The amino acid sequences of the antibodies were obtained by peptide sequencing in combination with mass-spectroscopic analysis. The sequences were confirmed by DNA sequencing of the cDNAs after cloning by reverse transcription and PCR amplification into suitable vector systems employing specific Ig primers combinations.

Protein sequencing was done from samples separated on an SDS-PAGE. For this an aliquot of sample was reduced with dithiothreitol, alkylated with iodoacetamide and subjected to SDS-PAGE using the NuPAGE system (Invitrogen). The gel was stained using InstantBluecoomassie stain (Expedeon Ltd) and bands representing heavy and light chains excised for mass spectrometric analysis.

### A LC-MS/MS

Each SDS-PAGE band was reduced with DTT, alkylated with iodoacetamide and subjected to proteolytic digestion using trypsin, chymotrypsin, or a sequential combination of both enzymes. The resulting peptides were separated by HPLC using an Ultimate 3000 nano-LC system (Dionex) equipped with a PepMap100 C18 column (75 m x 30 cm, Dionex) and analysed by tandem mass spectrometry using an amaZon ETD ion trap mass spectrometer (BrukerDaltonics) with electrospray ionisation. Spectral processing (Gaussian smoothing and baseline subtraction) and peak list generation (mgf format) was performed using DataAnalysis version 4.0 (BrukerDaltonics).

### B MALDI-MS/MS

A 1 µL aliquot of tryptic digest was spotted onto a MALDI target plate with cyano-4-hydroxy cinnamic acid as matrix. Positive-ion MALDI mass spectra (peptide mass fingerprints) were obtained using an ultraflex III (BrukerDaltonics) in reflectron mode, equipped with a Nd:YAG smart beam laser. Final mass spectra were externally calibrated against an adjacent spot containing six peptides of known mass. For each sample, the 10 strongest peaks, with a S/N greater than 35, were selected for MS/MS. Spectral processing (baseline subtraction and Savitsky-Golaysmoothing) and peak list generation (SNAP averagine algorithm; C 4.9384, N 1.3577, O 1.4773, S 0.0417, H 7.7583) were performed using flexAnalysis version 3.3 (BrukerDaltonics).

### C Mascot Database Searching (University of York)

All peak lists were submitted to database searching using a locally-running copy of the Mascot program (Matrix Science Ltd., version 2.1), through the ProteinScape interface (BrukerDaltonics., version 2.1). Searches were performed against the NCBInr database 20101130 restricted to mammalian sequences (856352 sequences), and subsequently against an in-house database containing the theorized potential antibody sequences. Search parameters specified: Enzyme; Trypsin, Chymotrypsin or No Enzyme, Peptide Mass Tolerance; 300 ppm, Fragment Mass Tolerance; 0.3 Da, Fixed Modifications; Carbamidomethyl (C), Variable Modification; Oxidation (M).

CD3 sequences were defined according to IMGT, the international ImMunoGene Tics information system and the PubMed NCBI database.

The sequences are listed in Figures 3 and 4

Following sequences were detected:
Consensus ACAB161 Variable domain Heavy Chain (Vh) sequence ACAB161 Vh 
EVMLVESGGGLVKPGGSLKLSCAASGFTFSRYTMSWVRQTPEKRLEWVATISSGGTNTNY-
TDSVKGRFTISRDNAKNNLYLQMSSLRSEDTALYYC-
TRGVRRGDGYAMDYWGQGTSVTVSSAKTTAPSVYPLAPVCGDTTGSSVTLGCLVK
(SEQ.ID.No.1)
 CDR3
   CTRGVRRGDGYAMDYW (SEQ.ID.No.11)
Consensus ACAB161 Variable domain Light Chain (Vl) sequence ACAB161 Vl 
DIVLTQAAPSVPVTPGESVSISCRSTKSLLHSNGNTYLYWFLQRPGQSPQALIYRTSNLAS-
GVPDRFSGSGSGTAFTLRISRVEAEDVGVYYCMQHLEYPLTFGAGTKLELKRADAAPTVS
(SEQ.ID.No.2)
 CDR3
   CMQHLEYPLTF (SEQ.ID.No.12)
Consensus ACAB164 Variable domain Heavy Chain (Vh) sequence ACAB164 Vh 
EVQLQQSGLALVQPGASVKISCRTSGYTFTEYTLHWVKQSHGKSLEWIGGINPN-
NGSPKYNQKFKGKATLTVDKSSSTAYMELRSLTSED-
SAVYYCARLDINSFFDYWGQGTTLTVSSAKTTPPSVYPLAPGCGDTTGSSVTLGCLVK
(SEQ.ID.No.3)
 CDR3
   CARLDINSFFDYW (SEQ.ID.No.13)
Consensus ACAB164 Variable domain Light Chain (Vl) sequence ACAB164 Vl 
DVVMTQSPLSLPVSLGDQASISCRSSQSLVHSNGNTYLQWYLQKPGQSPKLLIYKLS-
NRFSGVPDRFSGSGSGTDFTLKISRVEAEDLGVYSCSQTTHVPPTFGGGTKLEIKRADAAPTVS
(SEQ.ID.No.4)
 CDR3
   CSQTTHVPPTF (SEQ.ID.No.14)
ACAB167 Variable domain Heavy Chain (Vh) sequence ACAB167 Vh 
EVQLEESGGGLVQPKGSLKLSCTASGFPFNTYAMNWVRQAPGKGLEWVARIRTKSNNYATY-
YADSVKDRFTISRDDSQNMLYLQMNNLKTEDTAMYYCVRLYGYYFDYWGQGTTLTVSSAK-
TTPPSVYPLAP (SEQ.ID.No.5)
 CDR3
   CVRLYGYYFDYW (SEQ.ID.No.15)
ACAB167 Variable domain Light Chain (Vl) sequence ACAB167 Vl 
DIVMTQSPEIMSASPGEKVTMTCSASSSVSFMFWYQQKSGTSPKIWIYDTSKLAS-
GVPARFSGSGSGTSYSLTVSSMEAEDAATYYCQQWNSNPPTFGVGTKLELKRADAAPTVS
(SEQ.ID.No.6)
 CDR3
   CQQWNSNPPTF (SEQ.ID.No.16)
ACAB337 Heavy Chain sequence ACAB337 
DVQLQESGPGLVKP-
SQSLSLTCTVTGHSITSDYAWNWIRQFPGNKLEWVAYISYSGGTGYNPSLKSRISITRDTSKPN
QFFLQLNSVTTEDTATYYCACGDYYYGPRDYWGQGTSVTVSSAKTTPPSVYPLAP
(SEQ.ID.No.7)
 CDR3
   CGDYYYGPRDYW (SEQ.ID.No.17)
Consensus ACAB337 Variable domain Light Chain (Vl) sequence ACAB337 
DILLTQSPAILSVSPGERVSFSCRASQSIGTSIHWYQQRTNGSPRLLIKYASESIS-
GIPSRFSGSGSGTDFTLSINSVESEDIADYYCQQSNYWPFTFGSGTKLEIKRADAAPTVS
(SEQ.ID.No.8)
 CDR3
   CQQSNYWPF (SEQ.ID.No.18)
ACAB452 Heavy Chain sequence ACAB452 
DVQLQESGPGLVKPSQSLSLTCTVTGYSITSDYAWNWIRQFPGNKLEWVGYI
TYSGSTNYNPSLKSRISITRDTSKNQFFLQFNSVTSEDTATYFCARSWFAY-
WGQGTLVTVSSAKTTPPSVYPLAP (SEQ.ID.No.9)
 CDR3
   CARSWFAYW (SEQ.ID.No.19)
Consensus ACAB452 Variable domain Light Chain (Vl) sequence ACAB452 Vl 
DIVMTQTPLSLPVSLGDQASISCRSSQSLVHSNGNTYLHWFLQKPGQSPKLLI-
YKVSNRFSGVPDRFSGSGSGTDFTLKISRVEAEDLGVYFCLQSAHVPLTFGAG-
TKLELTRADAAPTVS (SEQ.ID.No.10)
 CDR3
   CLQSAHVPLTF (SEQ.ID.No.20)

### EXAMPLE 7: Specific IHC (immunohistochemistry) staining of intraepithelial neoplasia biopsies from the uterine cervix

The antibodies of the invention ("ACAB") were used to detect E7 protein expression in paraffin embedded tissues. A suitable method has been designed for the staining of tissues that have been fixed in neutral buffered formalin and subsequently embedded in paraffin before sectioning. Epitope retrieval pre-treatment of tissue samples was not required as all the ACAB allow for direct processing after rehydration in the staining protocol. Moreover the below procedure also applies for use in fully automatic IHC systems such as Ventana Systems (Benchmark Series XT) and Dako Systems (Dako Autostainer Plus). All protocol steps were performed at room temperature (RT, 22°C ± 4), unless otherwise indicated. To avoid damaging the slides, liquids were applied with care during the procedure. Staining jars were used
for immersing and rinsing slides and a humid chamber was used during incubation of the slides with Reagent 1, Reagent 2 (see Table 2) and secondary antibody.

**Table 2**

| | | pH |
|---|---|---|
| Reagent 1 | 3 %w/v BSA (Bovine Serum Albumin (fraction V)); phosphate buffered saline (PBS); 0.065% sodium azide (NaN₃) | 7.4 |
| Reagent 2 | A mixture of ACAB antibodies for some at concentrations used between 10mg/l to 20mg/l for most used between 1mg/l and 10mg/l; 0.1% w/v BSA (Bovine Serum Albumin (fraction V)); phosphate buffered saline (PBS); 0.065% sodium azide (NaN₃) | 7.4 |

### A Deparaffinization and rehydration

Prior to staining, tissue sections were deparaffinized and rehydrated. Slides were immersed in xylene for 30 minutes and excess liquid was removed. Slides were then successively immersed for 3 minutes each in ≥ 99.9%, 80% and 50% ethanol and then placed under running demineralized water for 3 minutes. The slice perimeters were marked with a liquid blocker pen (Super PAP Pen - Sigma cat. #Z672548-1EA) and slides were then immersed in PBS for 1 minute. Slides were allowed to drain and any excess liquid was removed. To eliminate endogenous peroxidases, slides were immersed in 0.3% hydrogen peroxide in PBS for 10 minutes and then immersed 3 times in PBS for 5 minutes. Any excess liquid was removed.

### B Primary antibody

To block slides, 250 µl of Reagent 1 (Table 2) was added to the section and slides were incubated in a humid chamber for 30 minutes. Slides were allowed to drain and excess liquid was removed. For primary antibody labeling, 250 µl of Reagent 2 (Table 2) was added to the slide, ensuring that the liquid was evenly spread within the PAP Pen marked region of the slide. Slides were incubated in a humid chamber for 1 hour, immersed 3 times for 3 minutes each in PBS + 0.1% Triton X100 and then rinsed with deionized water. Slides were allowed to drain and excess liquid was removed.

### C Secondary antibody incubation and DAB chromogenic development

For signal amplification and antibody visualization, commercially available immunoperoxidase systems were used. The Vectastain Universal Elite ABC Kit (cat. # PK-6200) or Universal Dako LSAB® Kit (code K0679/K0690) are working equally well. In any case the procedures were carried out according to the manufacturer's instructions. DAB chromogenic development was performed with the Vector DAB Peroxidase Substrate Kit (Vector Laboratories, cat. # SK-4100) according to the manufacturer's instructions.

Slides were counterstained with Modified Harris Hematoxylin solution (SIGMA, HHS32-1L; 10ml Hematoxylin + 70 ml dH₂O). Slides were then dehydrated by immersing successively for 3 minutes each in 50%, 80%, 96% and 100% ethanol and then for 3 minutes in xylene. Finally slides were mounted with Eukitt mounting medium. Samples were analyzed with a Leica DM2000 Microscope and 40x amplification.

### Results

FIGURE 5 A, B, C, D, most intraepithelial neoplasia biopsies from the uterine cervix detect the E7 antigen in the nucleus of the neoplastic cells. Fresh biopsies (A, C, D, E) as well as historical biopsies (F) of more 30 years of age can be stained with said ACAB's.

Some neoplastic cells do not show any E7 positive staining in a biopsy sample were as other neoplastic cells are equally stained. The E7 negative cells are positive for Haematoxilin staining and represent cells that are in a regressing state for E7 expression. Arrows show some examples for demonstration. Regression can be seen in basal as well as supra-basal cells.

### EXAMPLE 8: Immunocytochemistry used for staging of cervical cancer biopsies

The significance for the use of ACAB E7 IHC for the staging of intraepithelial lesions was analysed and compared to the surrogate marker INK4A. Biopsies with increasing disease stages were stained in parallel for E7 ACAB's and p16INK4A (MTM-Heidelberg) employing for both antigens the method suggested by the supplier for the p16INK4A. This protocol includes antigen retrieval and as such represents suboptimal conditions for E7 ACABs. Nevertheless results are clearly visible and comparable. Biopsies from the same tissue paraffin block were used.

### Results

FIGURE 6, all stages of intraepithelial neoplasia biopsies show a clear staining of the E7 antigen, indicative for E7 activity in the tissue biopsies analyzed. This is in contrast to the best described E7 surrogate marker p16INK4A that detects neoplastic cells more frequently with increasing staging. Whereas in early stages only E7 can be detected in later stages staining pattern are increasingly overlapping up to CIN2/3 were both patterns are super imposable. The latter indicates downstream activities for the E7 protein function. Double staining with one or more E7 surrogate marker can describe the carcinogenic activity of the analyzed tissue sample in a timely and disease stage dependent fashion.

### EXAMPLE 9: Cytofluorimetric analysis detects the expression of E7 in different cervical cancer isolates

### A Intracellular antibody labeling

For intracellular antibody labeling, 0.5 x 10⁶ cells were washed once with 1 ml ice-cold PBS. Cells were centrifuged at 350 x g for 5 minutes at 4°C and then fixed in 1% paraformaldehyde (1 ml/10⁶ cells) for 20 minutes at 4°C. Cells were washed with 1 ml ice-cold PBS and centrifuged at 350 x g for 5 minutes at 4°C. To permeabilize cells, 100% ice-cold methanol (1 ml/10⁶ cells) was added for 20 minutes at 4°C followed by centrifugation at 350 x g for 5 minutes at 4°C. Cells were washed with 1 ml ice-cold PBS, centrifuged at 350 x g for 5 minutes at 4°C and resuspended in 0.1% saponin + 2% FBS in PBS (ice-cold). Cells were left for 20-30 minutes at room temperature to reduce the background signal and then centrifuged at 350 x g for 5 minutes at 4°C. The primary antibody (ACAB; concentration 1 µg/µl; reference 25-77-1) was diluted in 0.1% saponin + 2% FBS (dilution 0.25 µg/50 µl of incubation buffer per 1 x 10⁶ cells). Cells were incubated with primary antibody for 30 minutes at room temperature, washed with 0.1% saponin + 2% FBS and centrifuged at 350 x g for 5 minutes at 4°C. The secondary antibody (FITC conjugated goat anti-mouse IgG1-IgM, BD, cat. # 555988) was diluted in 0.1% saponin + 2% FBS (dilution 1:50). Cells were incubated with secondary antibody for 30 minutes at room temperature, washed once with 0.1% saponin + 2% FBS, once with PBS and centrifuged at 350 x g for 5 minutes at 4°C.

### B Cytofluorimetric Analysis (CF)

Cells were resuspended in 700 µl PBS and then either analyzed by CF analysis or additional propidium iodide (PI) staining was performed to facilitate cell cycle analysis. For PI staining, cells were resuspended in 450 µl of PBS (per 10⁶ cells) and 100 µg/ml RNase (stock 1 mg/ml, in PBS) per 10⁶ cells was added for 10 minutes at 37°C. Cells were then incubated with 5 µg/ 10⁶ cells of PI (stock 1 mg/ml in PBS) for between 10 minutes and 2 hours at room temperature. Samples were stored at 4°C until CF analysis. Cytofluorimetric analysis were performed on an cytofluorimeter (Beckman Coulter FC 500 MPL, excitation laser 488nm). Data analysis was done using the Beckman Coulter software packages MXP for acquisition and CXP for data analysis , Detection ofHPV-E7 protein expression, was based on morphology back-gating of FL1 histogram (logarithmic scale) with gating for dot plot FS (Forward Scatter) log /SS (Sideward Scatter) log and gating for histograms SS log.

### EXAMPLE 10: E7 nuclear localisation analysed by cytofluorimetry

For the analysis of the E7 localisation in the cells a Cytofluorimetric Analysis of the entire cells and of the isolated nuclei was compared. This was done as described in EXAMPLE 9 and the nuclei were prepared introducing a detergent step in order to destroy the cell membranes, but leave the nuclear envelope intact.

The results are shown in Figure 8. Panel A (left) shows the auto-fluorescence background signal analyzing cells treated only with secondary antibody. Panel B (left) shows the total cell E7 fluorescence as evidenced by E7 specific antibodies of the invention. Panel B (right) shows the nuclear fluorescence and Panel A (right) shows the overlay of the blots in panel B. The arrows indicate the position of the auto-fluorescence background signal. These experiments demonstrate that in cycling cells, E7 is strongly enriched in the nucleus of the cell. The same result was obtained from the Immunofluorescence studies in Figure 13.

### EXAMPLE 11: Cytofluorimetric analysis of active E7 expressing tumor cells during the cell cycle

2x10⁷ Caski cells were grown for 24 hours in DMEM plus 10% FCS and then washed 2 times in DMEM 0.1% FCS, and serum starved for 48 hours (Time 0). Following serum starvation cells were released into the cell cycle by the addition of 20% FCS (serum-release). After different time points cells were prepared for cytofluorimetric Analysis as in EXAMPLE 9, using specific ACABs.

Results are shown in Figure 9. Whereas the E7 levels are low during serum starvation (A) E7 levels are increasing 1 hour after serum-release (B solid line, dotted line with arrow shows serum starvation level) and reaches its maximum intensity after 6 hours (C solid line, dotted line with arrow shows serum starvation level). For comparison the intensity after 1 hour serum release (dotted line arrow) is shown in an overlay with the 6 hour serum release as solid line (D). This example shows that E7 is constantly expressed during the cell cycle and shows lower protein levels in the resting phase of a cell.

### EXAMPLE 12: Cytofluorimetric analysis used for the screening of E7 antagonists

A Cytofluorimetric analysis in the presence of protein synthesis inhibitors was done. Steady state of the E7 protein was analyzed. These cells were grown and prepared for cytofluorimetric analysis using E7 specific staining as described in EXAMPLE 9 in the presence of the protein synthesis inhibitor cycloheximide (CyH). Samples were analysed after different time points. Higher signal (shifted to the right) indicates more E7 protein.

Results are shown in Figure 10. Panel A shows an overlay of cells prepared after 0, 0.5, 1, 2 and 6 hours after the addition of cycloheximide. The decrease in signal (shift to the left) indicates the inhibition of the new synthesis of E7 proteins. The individual blots are shown in panel B to G (B, no CyH; C, 0.5 hours CyH; D, 1 hour CyH; E, 2 hours CyH; F, 6 hours CyH; G, auto-fluorescence background signal; An assay can be designed using different molecules that inhibit E7 that leads to the degradation of E7 for the screening of drugs that are antagonists to E7. Isolated compounds can then be used to screen for compounds that inhibit cells with HPV E7 induced tumors and to a lesser extend non HPV dependent cells. These compounds can be further used to screen for compounds that directly interact with E7 proteins.

### EXAMPLE 13: E7 detection in Cytofluorimetry as a single, automatic read out assay for PAP testing

Cytofluorimetric (CF) analysis of E7 expressing in gynecological PAP or liquid PAP smear samples as a measure of LISIL and HSIL and ASC-US.

PAP smear samples can be used to be analyzed in cytofluorimetric analysis as described in EXAMPLE 9. For this liquid gynecological swaps were analyzed. Samples stored in most of the common transport media such as Cytec or Copan transport media proofed to be well suitable for this assay. Samples can be stored in liquid transport media before analysis for months without losing the E7 signal.

### Result

Individuals diagnosed with intraepithelial neoplasia from the uterine cervix show a significant increase in E7 specific signal on the Y axis (FL1), within the medium cell size population as measured by Sideward scattering on the X- axis (SS) indicating HSIL lesions of the individual. Figure 11 (A) shows samples from an individual diagnosed with HPV58 DNA positive HSIL/CIN3 and (C) shows samples from an individual diagnosed with HPV16 DNA positive HSIL/CIN3, (B + D) represent HPV negative samples, thus if one observes a staining as shown in (A+B) the individuals is diagnosed with intraepithelial neoplasia

### EXAMPLE 14: E7 specific signals in positive PAP smears

The ACAB-E7 antibodies are used for staining of gynecological PAP smear samples or liquid PAP smear samples fixed on a microscopic slide were stained for immunocytochemistry similar as described for EXAMPLE 7 except that step (A) was omitted, cells were fixed with 3% paraformaldehyde and then proceeded as described in EXAMPLE 7 "B Primary antibody".

### Results

The results show in Figure 12 panel (B) strong positive staining of neoplastic cells (arrow, brown). The sample shown was determined as HPV33 DNA positive, PAP classified HSIL. Panel A shows PAP negative samples stained with the same method. The result demonstrates that with the aid of the specific E7 staining using the ACABE7 of the invention the read out of a PAP result can be clearly confirmed by specific E7.

### Example 13: Predominant nuclear staining of E7 proteins in proliferating tumor cells

Caski cervical cancer cells were tested in immunofluorescence analysis using fluorescence marked secondary antibodies in a procedure similar to the one described for the ICC in EXAMPLE13 and reveal nuclear staining in the proliferating cells. This is an indication that active tumor cell progression contains E7 that is localized to the nucleus of the cells. This has important implications for analyzing IHC specimens and ICC specimens as nuclear localization indicates active tumor cells. These data confirm the cell cycle analysis and nuclear localization analysis with cyto-fluorimetry in EXAMPLE 11A and 11B. Figure 15 A shows the cells stained with E7 specific antibody and compares to B were total DNA stain with DAPI is shown.

### EXAMPLE 16: E7 specific Western blot detection of high and low copy number cancer cells

Extracts from proliferating cervical cancer cells were prepared and different aliquots corresponding to extract from different cell numbers were separated by SDS gel electrophoresis. Separated proteins were transferred to nitrocellulose membranes followed by western blot detection of E7 proteins, using the E7 specific ACAB161 as primary antibody. The high E7 copy Caski cell line containing more than 600 copies of E7 viral genomic DNA and the low E7 copy SiHa cell line containing only one copy E7 DNA was analyzed.

### Results

It was shown that in high E7 copy containing Caski cells E7 protein can be readily detected from extracts of only 1500 cells whereas in low copy SiHa cells weak detection of E7 was obtained with as little as 3000 cells. Semi-quantitative analysis reveals that Caski contain roughly about 40 times more E7 protein than SiHa cells that corresponds to 40/1 for E7 protein and does not reflect the difference in the E7 DNA content of 600/1. This is a clear demonstration that quantitative DNA analysis suggested for cervical cancer diagnostic purposes has only minor prognostic value for E7 levels at any stage of the carcinogenesis as it does not reflect the amount of causative oncoprotein E7 in a tumor cell.

The results are shown in Figure 14: A Caski cell extracts; B SiHa cell extracts; amount of cell extract loaded corresponding to the cell number of: 1=55000 cells, 2=25000 cells, 3=12500 cells, 4=6250 cells, 5=3000 cells, 6=1500 cells.

### EXAMPLE 17: Crossreactivity of the monoclonal E7 specific antibodies of the invention

Using ELISA and dot-blot techniques semiquantitative crossreactivities of the isolated mAbs was analysed. For this various mAbs were tested against the accepted high risk species. The results are summarized in Table 3. Importantly the high conservation of the primary amino acid sequences of the different species is also reflected in the crossreactivity of the mAbs of this invention. In average every antibody interacts at least with four HPVE7 types. The antibodies do not react with the beta-papillomavirus type proteins such as HPVE7 38 or HPV5, nor with the low risk type proteins HPV6 and HPV11. Figure 15 shows an exemplary result of an ELISA assay that detects crossreactivities summarized in Table 3.

**Table 3**

| | **16** | **18** | **31** | **33** | **35** | **39** | **45** | **51** | **52** | **56** | **58** | **59** |
|---|---|---|---|---|---|---|---|---|---|---|---|---|
| **ACAB161** | ++ | + | ++ | ++ | + | - | - | - | ++ | - | ++ | - |
| **ACAB164** | ++ | - | ++ | ++ | ++ | - | - | - | ++ | - | ++ | - |
| **ACAB167**** | ++ | - | ++ | ++ | - | na | - | - | ++ | - | ++ | - |
| **ACAB337*** | ++ | - | ++ | ++ | ++ | na | - | - | ++ | - | ++ | - |
| **ACAB452** | - | ++ | ++ | + | + | ++ | ++ | ++ | - | ++ | - | ++ |
| **ACAB4511** | + | ++ | - | - | - | ++ | ++ | ++ | - | ++ | - | ++ |

| | | | | | | | | | | | | |
|---|---|---|---|---|---|---|---|---|---|---|---|---|
| (++) strong positive; (+) weak interaction; (-) No interaction; (na) Not analysed First column ACAB antibodies; Top row numbers indicate E7 protein HPV type analysed; (*) indicates that for this antibody the antigens had to be denatured for detection (**) indicates that this antibody is not used in IHC | | | | | | | | | | | | |

### EXAMPLE 18: Surface Plasmon resonance

The kinetic constants kₒₙ and k_{off} were determined for ACABE7 antibodies binding to HPV-E7 antigens immobilized on a sensor chip using the BIAcore X100 (GE Healthcare, Uppsala, Sweden). For HPV-E7 immobilization HPV-E7 antigens prepared as in EXAMPLE 2 a Biotin moiety was coupled to primary amines by reductive amidation using Sulfo-NHS-LC Biotin (Pierce #21327) at a ratio of HPVE7:Biotin 1:5 at room temperature for 30 minutes and pH 6.0. Streptavidin immobilized sensor chip SA (GE Healthcare, Uppsala, Sweden) was than coated with the Biotin E7 proteins. Typically, as an example this was done by immobilizing 27RU of 1nM Biotin-HPV16E7 on Fc2, using the Wizard method and other 12RU in Manual run, using buffer containing 20mM Na₂HPO₄/NaH₂PO₄, 200mM NaCl, 0.005% Tween20. Typically 39RU were immobilized. Interaction between the SA chip bound biotinE7 proteins, and ACABE7 antibodies was analyzed using the antibody concentrations range of 1.56 - 400 nM. Repeated analysis was employed for estimating regeneration efficiency and reproducibility. Experimental conditions used were a flow rate of 60 µl/min at 25°C, injection/association phase was 90 seconds followed by a 600 seconds dissociation phase. Regeneration was performed with 10 mM glycine pH 2.5, 2 × 30 s at 10 µl/min, the stabilization period was 180 s. The experimental data were fitted to a bivalent analyte model. The residuals are shown below the sensorgrams. The Results are summarized in Table 4. Binders showed affinity range from between 213 and 5nM, most of the binders showed a KD of less than 10nM and some binders even showed a KD of less than 60 pM. Figure 16: provides exemplary Biacore kₒₙ and k_{off} curve fits for ACAB164.

**Table 4**

| **Antigen** | **mAB** | **Kₒₙ** | **K_{off}** | **KD M** |
|---|---|---|---|---|
| **16E7** | ACAB161 | 5, 842*10⁴ M⁻¹ s⁻¹ | 1.976*10⁻⁴ m⁻¹ s⁻¹ | 3.38*10⁻⁹ M |
| **16E7** | ACAB164 | 2.613*10⁵ M-1 s⁻¹ | 1.360*10⁻⁵ M⁻¹ s⁻¹ | 5.2*10⁻¹¹ M |
| **16E7** | ACAB167 | na | na | na |
| **33E7** | ACAB337 | nd | nd | nd |
| **45E7** | ACAB452 | 5.213*10⁴ M⁻¹ s⁻¹ | 5.514*10⁻⁴ M⁻¹ s⁻¹ | 10.6*10⁻⁹ M |
| **18E7** | ACAB452 | 9.467*10⁴ M⁻¹ s⁻¹ | 2.018*10⁻² M⁻¹ s⁻¹ | 213.0*10⁻⁹ M |
| **45E7** | ACAB4511 | 2.349*10⁵ M⁻¹ s⁻¹ | 1.119-10⁻⁷ m⁻¹ s⁻¹ | 4.8*10^{- 12}M |
| **18E7** | ACAB4511 | 2.167*10⁵ M⁻¹ s⁻¹ | 9.610*10⁻⁷ M⁻¹ s⁻¹ | 4.0*10^{- 12}M |

| | | | | |
|---|---|---|---|---|
| (na) not analysed, (nd) not detectable, indicates this mAb is not suitable for surface Plasmon resonance analysis | | | | |

### EXAMPLE 19: General description of an IHC kit according to the present invention: the CERVIMAX IHC kit

The CERVIMAX™ IHC monoclonal mouse antibody kit is used for qualitatively detecting HPV E7 protein expression in paraffin-embedded tissues or paraformaldehyde-immobilized cells by immunohistochemical staining.

It contains a monoclonal antibody against HPV 16 E7 raised according to the present invention.

The CERVIMAX™ IHC monoclonal antibody kit shows outstanding immunoreactivity for the detection of E7 antigen in formalin-fixed tissues. This makes it suitable for a direct application without antigen retrieval. In all experimental systems, E7 has been shown to be the prominent oncoprotein, exhibiting stronger malignant transforming effects than E6. Moreover, in contrast to E6, E7 is primarily localized in the nucleus during cell growth thus allowing for highly distinct staining patterns. Given its essential role in all stages of HPV-induced carcinogenesis, E7 makes an ideal target for research on HPV-induced cancer. As such, the presence of E7 proteins in human specimen samples may help to study malignant transformation and cancer.

The CERVIMAX™ IHC kit shows outstanding immunoreactivity with E7 antigen in paraffin-embedded tissues derived from low-grade squamous intraepithelial lesions, high-grade squamous intraepithelial lesions and invasive cancer. It shows consistently reliable performance on a wide range of tissue samples. The lack of robust functional monoclonal antibodies has hindered HPV research ever since E7 was identified as the primary oncogene in cervical cancer. The difficulty in detecting E7 proteins in clinical samples was commonly attributed to the low abundance and high instability of E7 proteins. However, the CERVIMAX™ IHC system shows that E7 is a robust and highly abundant antigen in clinical cervical cancer samples prepared under standardized protocols. With the CERVIMAX™ IHC kit, E7 proteins can be detected from fresh IHC samples and also from properly conserved historical paraffin-embedded samples that are more than 30 years old.

With the kit according to the present example, a reliable detection of E7 protein expression in paraffin-embedded tissues or paraformaldehyde-immobilized cells by immunohistochemical staining is possible. No antigen retrieval is required. Detection of high-risk E7 oncogenic proteins of the Alpha-Papillomavirus genus, species 5, 6, 7 and 9 is enabled. It is a ready-to-use kit and compatible with major immunohistochemical staining systems. It has superior sensitivity and a high signal-to-noise ratio. It is possible to have less than 4-hour turnover from sampling to result and is therefore well suited to routine testing.

It contains two reagents: Reagent 1 (Immunohistochemistry (IHC) Blocking Solution to reduce background staining by blocking excess binding sites on tissues; contains 0.065% Sodium Azide (NaN₃)) and Reagent 2, a stabilized solution of a mixture of ready-to-use mouse monoclonal antibodies (mAb) produced according to the present invention against high-risk HPV E7 oncogenic proteins. These monoclonal anti-E7 antibodies are purified from tissue culture supernatant and diluted in phosphate buffered saline (PBS) pH 7.4, containing 0.1% w/v Bovine Serum Albumin (fraction V) and 0.065% sodium azide (NaN₃).

The kit may further contain one or more of the following
components:
- Positively-charged microscope slides
- Slide drying chamber
- PAP pen for immunostaining
- Xylene or xylene substitute
- Peroxidase-based detection system containing biotinylated "Universal" secondary antibody for use with both rabbit and mouse primary antibodies (e.g. from Vector Laboratories, Vectastain Universal Elite Kit, cat. # PK-6200 or Universal Dako LSAB^{®} (Code K0679/K0690) or equivalent)
- Diaminobenzidine (DAB) (e.g. from Vector Laboratories, DAB Peroxidase Substrate Kit, 3,3'-diaminobenzidine, cat. # SK-4100 or equivalent)
- Reagent alcohol or ethyl alcohol
- Deionized water (dH₂O)
- Phosphate buffered saline (PBS) wash buffer, pH 7.6 + 0.1% Triton-X100
- Harris Modified Hematoxylin Counterstain (e.g. from Sigma, ACCUSTAIN^{®} HARRIS Hematoxylin Solution, cat. # HHS32)
- Timer
- Mounting medium
- Coverslips
- Staining jars and
- Humid Chamber.

CERVIMAX™ IHC makes it possible to visualize the distribution and localization of the E7 protein within the proper tissue context. The immunohistochemical detection steps include sample deparaffinization and rehydratation, elimination of endogenous peroxidases, antibody application, signal amplification and antibody visualization followed by optional counterstaining. Specimens are then coverslipped and observed by trained personnel using light microscopy.

CERVIMAX™ IHC kit has been designed for the staining of tissues that have been fixed in neutral buffered formalin and subsequently embedded in paraffin before sectioning.

All protocol steps should be performed at room temperature (RT, 22°C ± 4), unless otherwise indicated. To avoid damaging the slides, liquids should be applied with care during the procedure. Staining jars are recommended for immersing and rinsing slides. A humid chamber must be used during incubation of the slides with Reagent 1, Reagent 2 and secondary antibody.

Staining performance may be impaired if CERVIMAX™ IHC is used with detection systems or protocols other than those validated by the manufacturer. Variations in diluent, operator, pipetting technique, washing technique, incubation time, incubation temperature and kit age can alter staining performance. The condition and preparation of each tissue sample can affect results.

The CERVIMAX™ IHC staining procedure does not require any epitope retrieval pre-treatment of tissue samples and allows for direct processing after rehydration in the staining protocol.

Prior to staining, tissue sections must be deparaffinized to remove the embedding medium and rehydrated. Incomplete removal of paraffin may cause non-specific staining of the section.

For signal amplification and antibody visualization, CERVI-MAX™ IHC has been validated with commercially available immunoperoxidase systems. In particular, the following systems are recommended: Vectastain^{®} Universal Elite^{®} ABC Kit (cat. #PK-6200) and Universal Dako LSAB^{®} (Code K0679/K0690).

Other kit products with the same diagnostic aim (HPV E7) are e.g.

CERVIMAX™ ICC: a "Ready to use" system designed for the immunocytochemical detection of E7 antigen in gynecological samples and cells in culture.

CERVIMAX™ CF: a cytofluorometrical analysis of gynecological samples. The CF read out is "single factor" based.

CERVIMAX™ DIPSTICK: high quality CERVIMAX™ mAbs according to the present invention are used to develop Point-of-Care testing for fast and direct cervical cancer diagnosis using "dip-stick"-technology.

In the following, preferred embodiments of the present invention are described:
1. Method for preparing Human Papilloma Virus protein E7 antigen (HPV E7 antigen) comprising the following steps:
   - providing a purified preparation of HPV protein E7,
   - phosphorylating the HPV protein E7 in the preparation,
   - purifying the phosphorylated E7 protein with an anion exchange chromatography, wherein the phosphorylated E7 protein is separated from the non-phosphorylated E7 protein by a step wherein the non-phosphorylated E7 protein stays bound to an anion exchanger during the anion exchange chromatography whereas the phosphorylated E7 protein is obtained in the eluate of the anion exchange chromatography, thereby
   - obtaining a purified preparation of a phosphorylated HPV protein E7 antigen.
2. Method according to embodiment 1 wherein the purified preparation of HPV protein E7 is a recombinantly produced protein, preferably produced in E.coli, B. subtilis, S. cerevisiae P. pastoris, insect cells, plant cells, especially A.thaliana, N. tabacum or O. sativa or a mammalian host cell line, especially HEK293, CHO-K1 or CHO-WBL cells.
3. Method according to embodiment 1 or 2 wherein the purified preparation of HPV protein E7 contains more than 50 % HPV protein E7 per total protein, preferably more than 70 % HPV protein E7 per total protein, especially more than 90 % HPV protein E7 per total protein.
4. Method according to anyone of embodiments 1 to 3, wherein phosphorylation is performed at a protein concentration of 10 µg to 100 mg E7 protein per ml, preferably of 0.05 to 10 mg E7 protein per ml, especially of 0.1 to 5 mg E7 protein per ml.
5. Method according to anyone of embodiments 1 to 4, wherein phosphorylation is performed until at least 40%, preferably at least 80 %, especially at least 90 % of the E7 protein is fully phosphorylated.
6. Method according to anyone of embodiments 1 to 5, wherein phosphorylation is performed with a Casein Kinase (EC 2.7.11.1), preferably with Casein Kinase I or Casein Kinase II, especially recombinant Casein Kinase II, Casein Kinase II from rat liver or Casein Kinase II from calf thymus.
7. Method according to anyone of embodiments 1 to 6, wherein phosphorylation is performed at a temperature of 15 to 40°C, preferably from 20 to 37°C, and for a time of 10 min to 24 h, preferably from 30 min to 12 h, especially from 1 to 6 h.
8. Method according to anyone of embodiments 1 to 7, wherein phosphorylated HPV E7 protein is subjected to the anion exchange chromatography in a buffer having a ionic strength of 400mM NaCl or less, preferably of 250 mM NaCl or less, especially of 150 mM NaCl or less.
9. Method according to anyone of embodiments 1 to 8, wherein the anion exchange chromatography is performed with a beaded agarose or sepharose column with positively charged side chains, preferably a diethylaminoethyl, a quaternary aminoethyl or a quaternary ammonium agarose column, especially a Q Sepharose, MonoQ Sepharose CIM (Convective Interaction Media)-QA, CIM-DEAE or DEAE Sepharose anion exchange column.
10. Method according to anyone of embodiments 1 to 9, wherein the purified preparation of the phosphorylated HPV protein E7 antigen is dialysed, preferably at a temperature of 6 to 30°C, especially dialysed in the presence of Trehalose.
11. Human Papilloma Virus protein E7 antigen (HPV E7 antigen) preparation wherein the HPV E7 protein is fully phosphorylated and is essentially free of contaminating nucleic acids and lipids.
12. HPV E7 antigen preparation according to embodiment 11, wherein the E7 antigen is a recombinantly produced protein.
13. HPV E7 antigen preparation according to embodiment 11 or 12, wherein the E7 antigen contains a His-tag, a methionine residue, a serine residue at the N-terminus or at the C-terminus of the natural E7 protein sequence, especially a MHHHHHHS-peptide, a MHHHHHHSMSENLYFQGS-peptide or a GS-peptide at the N-terminus.
14. HPV E7 antigen preparation according to any one of embodiments 11 to 13, wherein the HPV E7 protein is selected from HPV 16, 18, 31, 33, 35, 39, 45, 51, 52, 56, 58, 59 and 66, preferably HPV 16, 18, 31, 33 and 45, especially HPV 16 and 18.
15. HPV E7 antigen preparation according to any one of embodiments 11 to 14, further comprising an adjuvant, preferably complete or incomplete Freund's adjuvant, aluminum salts (alum), especially aluminum hydroxide, aluminum phosphate, and aluminum sulphate; 3-De-O-acylated monophosphoryl lipid A (MPL), MF59, AS03, AS04, polysorbate 80 (Tween 80), imidazopyridine compounds, imidazoquinoxaline compounds, saponins, especially QS21; oil in water emulsions, especially comprising squalene or peanut oil; optionally in combination with immune stimulants, especially monophosphoryl lipid A, CpG, polymeric or monomeric amino acids, especially polyglutamic acid, polyarginine or polylysine.
16. HPV E7 antigen preparation according to any one of embodiments 11 to 15, wherein the preparation is in liquid form, preferably containing a buffer, especially a phosphate, carbonate or TRIS buffer.
17. HPV E7 antigen preparation according to any one of embodiments 11 to 16, further comprising a pharmaceutically acceptable carrier, preferably in a sterile pharmaceutical dosage form, especially as a vaccine.
18. HPV E7 antigen preparation according to any one of embodiments 11 to 17, wherein the preparation contains more than 40 % HPV protein E7 per total protein, preferably more than 70 % HPV protein E7 per total protein, especially more than 90 % HPV protein E7 per total protein.
19. HPV E7 antigen preparation according to any one of embodiments 11 to 18, wherein the antigen is obtained by a method according to any one of embodiments 1 to 10.
20. HPV E7 antigen preparation according to any one of embodiments 11 to 19, wherein trehalose is present.
21. A method for the generation of antibodies against HPV E7 antigen characterised by the following steps:
   - immunising mice, rats or rabbits, especially mice, with a HPV E7 antigen preparation according to any one of embodiments 11 to 20,
   - producing hybridomas from the immunised mice, and
   - screening the monoclonal antibodies produced by the hybridomas for binding ability to the HPV E7 antigen preparation according to any one of embodiments 11 to 20 and selecting those hybridomas that produce monoclonal antibodies having a binding ability to the HPV E7 antigen preparation according to any one of embodiments 11 to 20, and
   - generating antibodies against HPV E7 antigen from the selected hybridomas.
22. A method for the generation of antibodies against HPV E7 antigen characterised by the following steps:
   - contacting a HPV E7 antigen preparation according to any one of embodiments 11 to 20 with an antibody phage display or an antibody yeast display library in a soluble screening assay,
   - selecting those displayed antibodies that have a binding ability to the HPV E7 antigen preparation according to any one of embodiments 11 to 20 of 10⁻⁸ or less, and
   - isolating and optionally further expressing the antibodies that have a binding ability to the HPV E7 antigen preparation according to any one of embodiments 11 to 20 in a suitable expression system.
23. A method according to embodiment 21 or 22, wherein the generated antibodies are purified and optionally finalised to a diagnostical or pharmaceutical preparation.
24. A method according to any one of embodiments 21 to 23, wherein the antibodies are provided as IgM or IgG antibodies, especially IgG antibodies.
25. A method for the generation of polyclonal antibodies against HPV E7 antigen characterised by the following steps:
   - eliciting an in-vivo humoral response in a non-human animal, preferably a mammal, especially a mouse, a rat or a rabbit against a HPV E7 antigen preparation according to any one of embodiments 11 to 20,
   - purifying, especially affinity-purifying, antibodies obtained in the eliciting step that bind to a HPV E7 antigen preparation according to any one of embodiments 11 to 20.
26. Antibody specifically recognising a HPV E7 antigen preparation according to any one of embodiments 11 to 20.
27. Antibody according to embodiment 26, characterised in that it is a monoclonal antibody.
28. Antibody according to embodiment 26 or 27, characterised in that it is an IgM or IgG antibody, especially an IgG antibody.
29. Antibody according to any one of embodiments 26 to 28, characterised in that it is a murine, a humanised or a human antibody; a full length antibody, a single chain Fv fragment, a single chain Fv2 fragment, a minibody, a bispecific antibody, a diabody, a triabody, a tetrabody, a di-diabody, a bispecific minibody, a tribiminibody, a Camelid antibody, a Shark antibody, a bispecific single chain Fv fragment, a Fab₂, or a Fab₃.
30. Antibody according to any one of embodiments 26 to 29, characterised in that it was obtained by a method according to any one of embodiments 21 to 25.
31. Antibody according to any one of embodiments 26 to 30, characterised in that it has a specific affinity K_{off} for a HPV E7 antigen preparation according to any one of embodiments 11 to 20 of 10⁻⁷ or less, preferably of 10⁻⁸ or less, especially of 10⁻⁹ or less.
32. Antibody according to any one of embodiments 26 to 31, characterised in that it comprises an amino acid sequence according to SEQ.ID.NO. X to XX.
33. Antibody according to any one of embodiments 26 to 32, characterised in that it is an HPV protein E7 binding antibody fragment, preferably a Fab, Fv, scFv, F(ab')₂ and Fd fragment.
34. Antibody according to any one of embodiments 26 to 33, characterised in that it is a chimeric antibody, a humanised antibody or a single-chain antibody.
35. Antibody according to any one of embodiments 26 to 34, characterised in that it is conjugated, preferably covalently conjugated, with a label, preferably a radioisotope, an enzyme which generates a detectable product, a fluorescent protein, biotin or avidin; or with a solid support, preferably polystyrene plates or beads, glass slides or beads.
36. Antibody according to any one of embodiments 26 to 35, characterised in that it is conjugated, preferably covalently conjugated, with a small molecule anti cancer drug, especially natural and synthetic tubulysins, natural and synthetic epithelons, antracyclins, auristatins or mesothelins.
37. Antibody according to any one of embodiments 26 to 36, characterised in that it detects the HPV protein E7 in the nucleus of human cells.
38. Antibody according to any one of embodiments 26 to 37, characterised in that it specifically recognises native HPV protein E7 in tissue specimen of HPV infected tissue.
39. Antibody according to any one of embodiments 26 to 38, characterised in that it specifically recognises a HPV 16 E7 antigen preparation according to any one of embodiments 11 to 20.
40. Antibody according to any one of embodiments 26 to 39, characterised in that it is an IgG antibody.
41. Preparation of an antibody according to any one of embodiments 26 to 40 comprising the antibody and a diagnostically or pharmaceutically acceptable carrier, diluent, buffer or storage solution, preferably in a sterile or sterilised state.
42. Method for in vitro detecting HPV protein E7 in a biological sample, wherein an antibody according to any one of embodiments 26 to 40 is used for binding protein E7 in the sample.
43. Method according to embodiment 42, wherein the biological sample is a Pap-smear, a cervical scrape, a cervical lavage, a cervical (carcinoma) biopsy, a mucosal specimen, an anogenital biopsy, a mamma biopsy, a head-or neck biopsy, a bladder biopsy, a colon cancer biopsy, a lung cancer biopsy or a prostate biopsy; a human blood, serum or plasma sample, human saliva or human urine.
44. Method according to embodiment 42 or 43, comprising the following steps:
   - incubating a biological sample with an antibody according to any one of embodiments 26 to 40 and
   - measuring and/or detecting specific binding of the antibody to HPV protein E7 in the biological sample.
45. Method according to any one of embodiments 42 to 44 for diagnosing a disease, preferably for diagnosing a sexually transmittable disease, a HPV16-infection cervical cancer, breast cancer/mamma cancer, prostate cancer, bladder cancer, colon cancer, lung cancer, head and neck cancer, penil cancer and/or anogenital cancer/neoplasia (AIN).
46. Method according to any one of embodiments 42 to 45, comprising the following steps:
   - incubating the sample with a monoclonal antibody according to any one of embodiments 26 to 40,
   - contacting the monoclonal antibody that is bound to the HPV protein E7 with a second antibody that binds to the monoclonal antibody; wherein the second antibody is conjugated to a detectable compound and
   - detecting binding of the second antibody, thereby detecting the HPV protein E7.
47. Method according to any one of embodiments 42 to 46, wherein the HPV protein E7 is HPV 16 protein E7.
48. Method according to any one of embodiments 42 to 47, wherein the method is selected from immune histochemistry (IHC), immune cytochemistry (ICC), cytofluorimetry (CF), immunofluorescence (IF), enzyme-linked immunosorbent assay (ELISA), Western blot (WB) or dip-stick testing.
49. Method according to any one of embodiments 42 to 48, wherein the method comprises an automatic staining and/or automatic read-out step.
50. Method according to any one of embodiments 42 to 49, wherein the method comprises the testing of another marker for HPV and/or a tumour.
51. Kit for in vitro detecting HPV protein E7 in a biological sample, comprising an antibody according to any one of embodiments 26 to 40.
52. Kit according to embodiment 51, further comprising a device wherein the biological sample is contacted with the antibody.
53. Kit according to embodiment 51 or 52, further comprising a second antibody for detecting the antibody according to any one of embodiments 26 to 40, preferably the second antibody comprises a label, especially a fluorescent, chromogenic, magnetic or radioactive label.
54. Kit according to any one of embodiments 51 to 53, further comprising reagents for detecting the antibody according to any one of embodiments 26 to 40 by an enzyme-linked immunosorbent assay.
55. Kit according to any one of embodiments 51 to 54, wherein the antibody according to any one of embodiments 26 to 40 is contained in an immobilised form.
56. Kit according to any one of embodiments 51 to 55, further comprising standard samples for HPV protein E7.
57. Kit according to any one of embodiments 51 to 56, wherein the antibody according to any one of embodiments 26 to 40 is detecting HPV 16 E7 antigen.
58. Kit according to any one of embodiments 51 to 57, further comprising positive and/or negative samples.
59. Kit according to any one of embodiments 51 to 58, wherein the kit is an immune histochemistry (IHC) kit, an immune cytochemistry (ICC) kit, a cytofluorimetry (CF) kit, immunofluorescence (IF), enzyme-linked immunosorbent assay (ELISA), Western blot (WB) or a dip-stick testing kit.
60. Kit according to any one of embodiments 51 to 59, wherein the kit further comprises means for automatic staining and/or automatic read-out.

## Claims

1. Method for preparing Human Papilloma Virus protein E7 antigen (HPV E7 antigen) comprising the following steps:
- providing a purified preparation of HPV protein E7,
- phosphorylating the HPV protein E7 in the preparation,
- purifying the phosphorylated E7 protein with an anion exchange chromatography, wherein the phosphorylated E7 protein is separated from the non-phosphorylated E7 protein by a step wherein the non-phosphorylated E7 protein stays bound to an anion exchanger during the anion exchange chromatography whereas the phosphorylated E7 protein is obtained in the eluate of the anion exchange chromatography, thereby
- obtaining a purified preparation of a phosphorylated HPV protein E7 antigen.

2. Method according to claim 1, wherein phosphorylation is performed with a Casein Kinase (EC 2.7.11.1), preferably with Casein Kinase I or Casein Kinase II, especially recombinant Casein Kinase II, Casein Kinase II from rat liver or Casein Kinase II from calf thymus.

3. Method according to claim 1 or 2, wherein phosphorylated HPV E7 protein is subjected to the anion exchange chromatography in a buffer having a ionic strength of 400mM NaCl or less, preferably of 250 mM NaCl or less, especially of 150 mM NaCl or less.

4. Method according to anyone of claims 1 to 3, wherein the anion exchange chromatography is performed with a beaded agarose or sepharose column with positively charged side chains, preferably a diethylaminoethyl, a quaternary aminoethyl or a quaternary ammonium agarose column, especially a Q Sepharose, MonoQ Sepharose CIM (Convective Interaction Media)-QA, CIM-DEAE or DE-AE Sepharose anion exchange column.

5. Human Papilloma Virus protein E7 antigen (HPV E7 antigen) preparation wherein the HPV E7 protein is fully phosphorylated and is essentially free of contaminating nucleic acids and lipids.

6. HPV E7 antigen preparation according to claim 5, wherein the E7 antigen is a recombinantly produced protein.

7. HPV E7 antigen preparation according to claim 5 or 6, wherein the HPV E7 protein is selected from HPV 16, 18, 31, 33, 35, 39, 45, 51, 52, 56, 58, 59 and 66, preferably HPV 16, 18, 31, 33 and 45, especially HPV 16 and 18.

8. HPV E7 antigen preparation according to any one of claims 5 to 7, further comprising a pharmaceutically acceptable carrier, preferably in a sterile pharmaceutical dosage form, especially as a vaccine.

9. HPV E7 antigen preparation according to any one of claims 5 to 8, wherein the antigen is obtained by a method according to any one of claims 1 to 4.

10. A method for the generation of antibodies against HPV E7 antigen **characterised by** the following steps:
- immunising mice, rats or rabbits, especially mice, with a HPV E7 antigen preparation according to any one of claims 5 to 9,
- producing hybridomas from the immunised mice, and
- screening the monoclonal antibodies produced by the hybridomas for binding ability to the HPV E7 antigen preparation according to the present invention and selecting those hybridomas that produce monoclonal antibodies having a binding ability to the HPV E7 antigen preparation according to the present invention, and
- generating antibodies against HPV E7 antigen from the selected hybridomas.

11. A method for the generation of antibodies against HPV E7 antigen **characterised by** the following steps:
- contacting a HPV E7 antigen preparation according to any one of claims 5 to 9 with an antibody phage display or an antibody yeast display library in a soluble screening assay,
- selecting those displayed antibodies that have a binding ability to the HPV E7 antigen preparation according to any one of claims 5 to 9 of 10⁻⁸ or less, and
- isolating and optionally further expressing the antibodies that have a binding ability to the HPV E7 antigen preparation according to any one of claims 5 to 9 in a suitable expression system.

12. A method for the generation of polyclonal antibodies against HPV E7 antigen **characterised by** the following steps:
- eliciting an in-vivo humoral response in a non-human animal, preferably a mammal, especially a mouse, a rat or a rabbit against a HPV E7 antigen preparation according to any one of claims 5 to 9,
- purifying, especially affinity-purifying, antibodies obtained in the eliciting step that bind to a HPV E7 antigen preparation according to any one of claims 5 to 9.

13. Antibody specifically recognising a HPV E7 antigen preparation according to any one of claims 5 to 9, preferably a monoclonal antibody, especially an IgG antibody.

14. Method for in vitro detecting HPV protein E7 in a biological sample, wherein an antibody according to claim 13 is used for binding protein E7 in the sample.

15. Kit for in vitro detecting HPV protein E7 in a biological sample, comprising an antibody according to claim 13.
